(19) Europäisches Patentamt
European Patent Office
Office européen des brevets

(11) Numéro de publication: **0 210 929 B1**

(12) # FASCICULE DE BREVET EUROPEEN

(45) Date de publication de fascicule du brevet: **03.06.92**

(51) Int. Cl.[5]: **C07C 63/331**, C07C 63/49, C07C 65/24, C07D 307/68, C07D 333/40, C07D 311/92

(21) Numéro de dépôt: **86401671.2**

(22) Date de dépôt: **25.07.86**

(54) **Dérivés aromatiques polycycliques, leur procédé de préparation et leur application dans les domaines pharmaceutique et cosmetique.**

(30) Priorité: **25.07.85 LU 86022**

(43) Date de publication de la demande:
**04.02.87 Bulletin 87/06**

(45) Mention de la délivrance du brevet:
**03.06.92 Bulletin 92/23**

(84) Etats contractants désignés:
**AT BE CH DE FR GB IT LI NL SE**

(56) Documents cités:

**JOURNAL FÜR PRAKTISCHE CHEMIE, vol. 318, no. 5, 1976, pages 731-744, J.A. Barth, Leipzig; J. LIEBSCHER et al.: "Synthesen von 2,5-disubstituierten Thiophenen aus 1-Chlor-propenyliden-(3)-immoniumsalzen und Schwefel-Nucleophilen"**

(73) Titulaire: **CENTRE INTERNATIONAL DE RECHERCHES DERMATOLOGIOUES GALDERMA - CIRD GALDERMA**
**Sophia Antipolis**
**F-06560 Valbonne(FR)**

(72) Inventeur: **Shroot, Braham Villa 35**
**Hameaux de Val Bosquet Chemin de Val Bosquet**
**F-06600 Antibes(FR)**
Inventeur: **Eustache, Jacques**
**42, avenue Alphonse Daudet**
**F-06130 Grasse(FR)**
Inventeur: **Watts, Oliver**
**"Les roses de France" 59, avenue du Val Fleuri**
**F-06800 Cagnes S/Mer(FR)**
Inventeur: **Bernardon, Jean-Michel**
**21, Chemin Plan Bergier Le Rouret**
**F-06650 Nice(FR)**
Inventeur: **Nedoncelle, Philippe**
**10, Boulevard Emile Zola**
**F-06130 Grasse(FR)**

EP 0 210 929 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

**BULLETIN DE LA SOCIETE CHIMIOUE DE FRANCE, partie 2, novembre/décembre 1975, pages 2521-2526; K. CHEBAANE et al.: "Synthèse d'aryl-2 naphtalènes et de dibenzocoumarines. 2ème partie: Aromatisation par le palladium/charbon des tétrahydrodibenzocoumarines: Synthèse d'aryl-2 naphtalènes et de dibenzocoumarines"**

(74) Mandataire: **Nony, Michel et al**
**Cabinet NONY & CIE, 29, rue Cambacérès**
**F-75008 Paris(FR)**

## Description

La présente invention a pour objet des dérivés aromatiques polycycliques, leur procédé de préparation et leur utilisation dans les domaines thérapeutique et cosmétique.

Ces dérivés aromatiques polycycliques trouvent une application dans le traitement topique et systémique des affections dermatologiques liées à un désordre de la kératinisation (différenciation-prolifération) et d'affections dermatologiques, ou autres, à composantes inflammatoires et/ou immunoallergiques et-dans le traitement des maladies de dégénérescence du tissu conjonctif, ainsi qu'une activité antitumorale. En outre, ces dérivés peuvent être utilisés dans le traitement de l'atopie, qu'elle soit cutanée ou respiratoire et du psoriasis rhumatoïde.

Ils trouvent également une application dans le domaine ophtalmologique, notamment pour le traitement des cornéopathies.

L'état de la technique concernant les composés selon l'invention est essentiellement représenté par le document Journal Für Praktische Chemie Vol.318 n° 5, 731-744, 1976.

Les dérivés aromatiques polycycliques selon l'invention peuvent être représentés par la formule générale suivante :

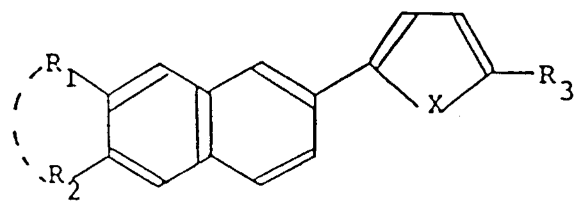

(I)

dans laquelle :

X représente -CH = CH-, 0 ou S,

$R_1$ et $R_2$, identiques ou différents, représentent un atome d'hydrogène, un radical alkyle, linéaire ou ramifié, ayant de 1 à 15 atomes de carbone, un radical alkoxy ayant de 1 à 6 atomes de carbone, un radical 1-adamantyle, ou $R_1$ et $R_2$, pris ensemble, forment avec les atomes de carbone adjacents du noyau naphtalénique, un cycle à 5 ou 6 chaînons, éventuellement substitué(s) par au moins un radical alkyle inférieur, ou interrompu par un atome d'oxygène,

$R_3$ représente le radical $-CH_2OH$, ou $-COR_4$ ou encore le radical $-CH_3$ lorsque $R_1$ et $R_2$, pris ensemble, forment un cycle à 5 ou 6 chaînons,

$R_4$ représentant $-OR_5$ ou

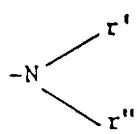

$R_5$ représentant un atome d'hydrogène, un radical alkyle ayant 1 à 20 atomes de carbone , monohydroxyalkyle, polyhydroxyalkyle, aryle ou aralkyle ou un reste d'un sucre ou encore représente le radical :

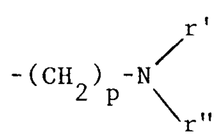

p étant 1, 2 ou 3,

r' et r'' représentant un atome d'hydrogène, un radical alkyle inférieur, un radical monohydroxyalkyle ou polyhydroxyalkyle, un radical aryle éventuellement substitue par un groupe hydroxy ou un reste d'aminoacide ou de sucre aminé ou encore pris ensemble forment un hétérocycle,

ou encore X, $R_1$ et $R_2$ représentent respectivement -CH = CH-, 1-adamantyl et -OH et $R_3$ représente -COOH

ou $-COOCH_3$,

sous réserve que $R_1$ et $R_2$ ne peuvent simultanément représenter un atome d'hydrogène lorsque X représente un atome de soufre et $R_3$ le radical $-COOCH_3$,

et les sels desdits dérivés aromatiques polycycliques de formule(I).

Par radical alkyle ayant de 1 à 20 atomes de carbone on doit entendre notamment les radicaux méthyle, éthyle, propyle,2- éthyl-hexyle, octyle, dodécyle, hexadécyle et octadécyle.

Par radical alkyle inférieur on doit entendre un radical ayant de 1 à 4 atomes de carbone notamment les radicaux méthyle, éthyle, isopropyle, butyle et tertiobutyle.

Par radical monohydroxyalkyle, on doit entendre un radical ayant 2 ou 3 atomes de carbone, notamment un radical 2- hydroxy éthyle ou 2-hydroxy propyle.

Par radical polyhydroxyalkyle, on doit entendre un radical contenant de 3 à 6 atomes de carbone et de 2 à 5 groupes hydroxyles tels que les radicaux 2,3- dihydroxy propyle, 2,3,4-trihydroxy butyle, 2,3,4,5-tétrahydroxy pentyle ou le reste du pentaérythritol.

Par reste d'un sucre, on doit entendre un reste dérivant par exemple du glucose, du mannose ou de l'érythrose ou du galactose.

Parmi les restes de sucres aminés on peut citer ceux dérivant de glucosamine, de galactosamine, ou de mannosamine.

Parmi les radicaux alkoxy ayant de 1 à 6 atomes de carbone on peut citer le radical méthoxy, isopropoxy et tert-butoxy.

Lorsque les radicaux r' et r'' pris ensemble forment un hétérocycle, celui-ci est de préférence un radical pipéridino, pipérazino, morpholino ou pyrrolidino.

Lorsque les composés selon l'invention se présentent sous forme de sels, il peut s'agir soit de sels d'un métal alcalin ou alcalino-terreux ou encore de zinc, ou d'une amine organique lorsqu'ils comportent au moins une fonction acide libre ($R_3 = COOH$), soit de sels d'un acide minéral ou organique, notamment de chlorhydrate, de bromhydrate ou de citrate lorsque qu'ils comportent au moins une fonction amine.

Parmi les composés de formule (I) particulièrement préférés selon l'invention, on peut mentionner ceux dans lesquels $R_3 = -COOR_5$ et plus particulièrement correspondant aux formules suivantes :

(A) 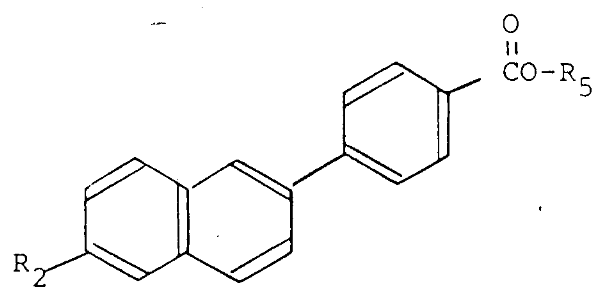 (II)

dans laquelle :

$R_5$ représente un atome d'hydrogène ou un radical alkyle

et

$R_2$ représente un radical alkyle inférieur ramifié.

(B) 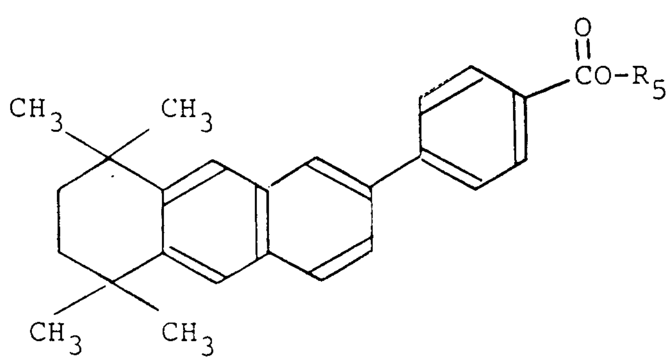 (III)

dans laquelle :

$R_5$ représente un atome d'hydrogène ou un radical alkyle.

(C) 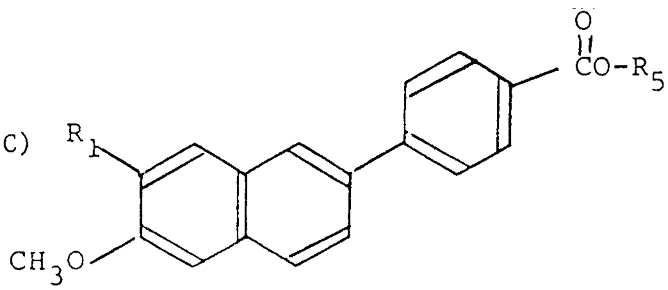 (IV)

dans laquelle:

$R_1$ représente un radical tert-butyle ou 1-adamantyle,

et

$R_5$ représente un atome d'hydrogène ou un radical alkyle.

(D) 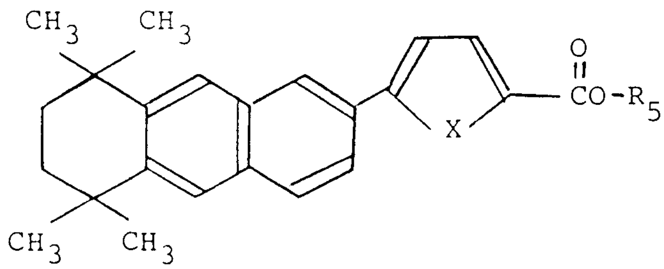 (V)

dans laquelle:

X représente 0 ou S,

et

$R_5$ représente un atome d'hydrogène ou un radical alkyle.

Parmi les composés de formule (I) on peut notamment citer les suivants :

- l'acide p-(6-tert-butyl-2-naphtyl) benzoïque,
- l'acide p-(5,6,7,8 tétrahydro-5,5,8,8-tétraméthyl-2-anthracényl) benzoïque et ses esters éthylique, 2-hydroxyéthylique et 2,3-dihydroxypropylique,
- le diéthylamide de l'acide p-(6-tert-butyl-2naphtyl)benzoïque,
- le monoéthylamide de l'acide p-(5,6,7,8-tétrahydro-5,5,8,8-tétraméthyl-2-anthracényl)-benzoïque,
- l'acide p-(6-méthoxy-2-naphtyl)-benzoïque et son ester méthylique,
- l'alcool p-(5,6,7,8-tétrahydro-5,5,8,8-tétraméthyl-2-anthracényl)-benzylique,
- le 2-(p-méthylphényl)-5,6,7,8-tétrahydro-5,5,8,8-tétraméthyl-anthracène,
- le p-hydroxyphénylamide de l'acide p-(5,6,7,8-tétrahydro-5,5,8,8-tétraméthyl-2-anthracényl)-benzoïque,
- l'acide p-[7-(1-adamantyl)-6-méthoxy-2 naphtyl]-benzoïque et son ester méthylique,
- l'acide p-(7-tert-butyl-6-méthoxy-2-naphtyl)-benzoïque et son ester méthylique,
- l'acide p-[7-(1-adamantyl)-6 hydroxy-2-naphtyl]-benzoïque et son ester méthylique,
- l'acide 5-(5,6,7,8-tétrahydro-5,5,8,8-tétraméthyl-2-anthracényl)-2-furanne carboxylique et son ester méthylique,
- l'acide 5-(5,6,7,8-tétrahydro-5,5,8,8-tétraméthyl-2-anthracényl)-2-thiophène carboxylique et son ester méthylique,
- l'éthylamide de l'acide 5-(5,6,7,8-tétrahydro-5,5,8,8 tétraméthyl-2-anthracényl)-2 furanne carboxylique,
- le morpholide de l'acide 5-(5,6,7,8-tétrahydro-5,5,8,8-tétraméthyl-2 anthracényl)-2 furanne carboxylique,
- le 5-(5,6,7,8-tétrahydro-5,5,8,8-tétraméthyl-2-anthracényl)-2-furanne carboxylate de 2-hydroxyéthyle,
- l'acide p-[3,4(2H)-dihydro-4,4-diméthyl-7 naphto (2,3-b) pyrannyl]-benzoïque et son ester méthylique,

Bien que diverses méthodes de synthèse puissent être envisagées pour l'obtention des composés de formule (I) on préfère utiliser selon l'invention la méthode représentée par le schéma réactionnel suivant :

Schéma A

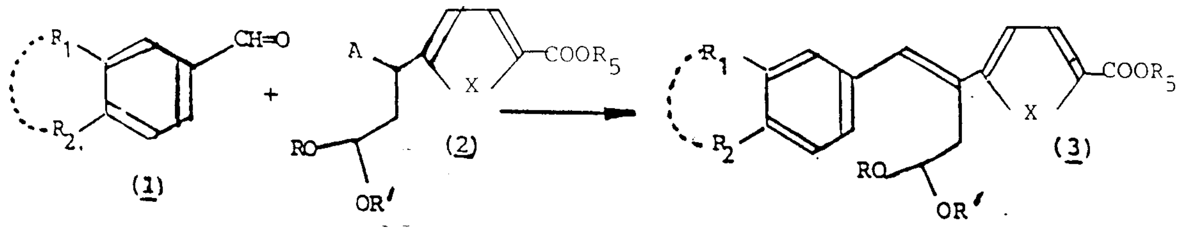

(1) (2) (3)

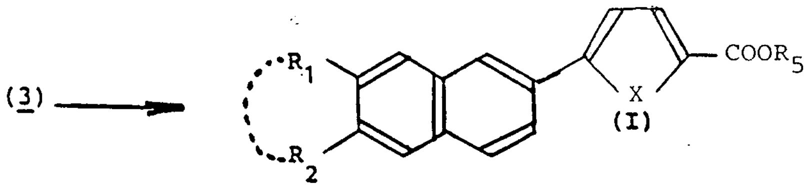

(I)

R et R' représentant un radical alkyle inférieur ou pris ensemble forment un cycle dioxanne ou dioxolane.

Selon cette méthode, on effectue tout d'abord une réaction de couplage du type Wittig ou Wittig-Horner entre un aldéhyde aromatique (1) et un dérivé de phosphore pentavalent (2).

Dans le dérivé (2) le radical A peut représenter soit un groupement triaryl phosphonium de formule : -P [X']$_3^{\oplus}$ , Y$^{\ominus}$, X' étant un aryle et Y un anion d'un acide organique ou inorganique, soit un groupement dialcoxyphosphinyle de formule:

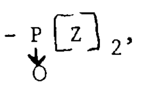

Z étant un alkoxy, de préférence $-OC_2H_5$.

Lorsque A représente -P[X']$_3^{\oplus}$ Y$^{\ominus}$ , la réaction de couplage est effectuée en présence d'un alcoolate de métal alcalin, tel que le méthylate de sodium ou en présence d'un oxyde d'alcoylène éventuellement substitué par un groupe alcoyle, dans un solvant tel que le chlorure de méthylène ou le diméthylformamide. La température de réaction est comprise entre 0 ° C et la température d'ébullition du mélange réactionnel.

Lorsque A représente

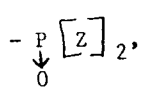

la condensation est effectuée en présence d'une base et de préférence en présence d'un solvant organique inerte, par exemple au moyen d'hydrure de sodium ou d'un dialkylamidure de lithium dans du benzène, du toluène, du diméthylformamide(dans le cas de l'hydrure de sodium), du tétrahydrofuranne, du dioxane ou du 1,2-diméthoxy éthane ou également au moyen d'un alcoolate, par exemple au moyen de méthylate de sodium dans le méthanol ou de tert-butylate de potassium dans le tétrahydrofuranne (T.H.F), à une température comprise entre -80 ° C et le point d'ébullition du mélange réactionnel. La condensation peut être également réalisée en utilisant une base minérale, telle que la potasse ou la soude, dans un solvant organique tel que le tétrahydrofuranne ou dans des conditions de transfert de phases.

On peut ajouter au mélange réactionnel un éther couronne susceptible de complexer le cation métallique contenu dans la base, ce qui permet d'augmenter la force de celle-ci.

Le composé intermédiaire de formule (3) est généralement obtenu sous forme d'un mélange d'isomères trans (E) et cis (Z) que l'on peut séparer par chromatographie, mais le mélange d'isomères peut être utilisé tel quel pour l'étape suivante.

La réaction de cyclisation-aromatisation est réalisée dans un solvant chloré tel que le dichlorométhane ou le chloroforme en présence, comme catalyseur, d'un acide fort tel que l'acide sulfurique ou l'acide p-toluène sulfonique ou un ester silylique d'un acide fort tel que par exemple le trifluorométhanesulfonate de

triméthylsilyle (TMSOTF).

Lorsque l'on utilise l'isomère (Z) du composé de formule (3) ou un mélange d'isomères (E)-(Z), la réaction de cyclisation-aromatisation doit être conduite sous irradiation UV afin d'isomériser l'isomère (Z) en son isomère (E).

En effet, dans les conditions de la réaction, les isomères (Z) ne conduisent pas aux composés attendus de formule (I).

Cette réaction de cyclisation-aromatisation est de préférence conduite à température ambiante.

Les aldéhydes aromatiques de formule (1) sont soit disponibles dans le commerce, soit facilement accessibles par les méthodes connues de synthèse.

La préparation du dérivé de phosphore pentavalent de formule (2) peut être réalisée selon le schéma réactionnel suivant :

SCHEMA B

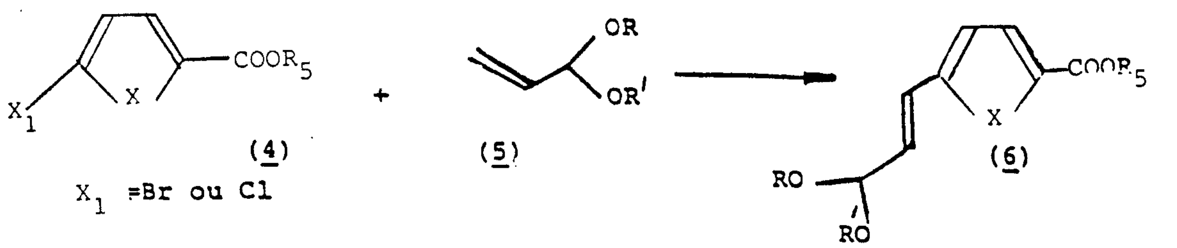

(4) (5) (6)

$X_1$ =Br ou Cl

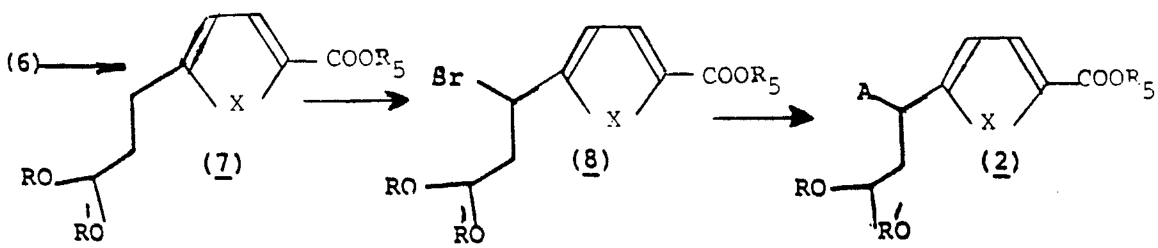

(7) (8) (2)

Selon cette méthode on réalise tout d'abord une réaction de couplage entre un halogéno ester d'alkyle (4) et un dialkylacétal de l'acroléine en présence d'un sel de palladium et d'une base telle que la triéthylamine, la di-isopropylamine, le bicarbonate de sodium ou le carbonate de sodium, la température étant de préférence comprise entre 70 et 150 °C. Le composé intermédiaire (6) est ensuite hydrogéné en composé de formule (7) puis bromé par le n-bromosuccinimide dans le tétrachlorure de carbone pour conduire au composé bromé (8). Ce dernier est ensuite transformé en dérivé de phosphore pentavalent de formule (2) par exemple à l'aide d'un phosphite organique tel que le triéthyl phosphite selon les conditions de la réaction d'ARBUSOV.

Lorsque les composés de formule (I), selon l'invention, sont des dérivés naphtaléniques mono-substitués ($R_1$ ou $R_2$ = alkyle $C_1$-$C_{15}$) et plus particulièrement des composés de formule (II), on préfère utiliser le procédé faisant intervenir les étapes représentées par le schéma réactionnel suivant :

SCHEMA C

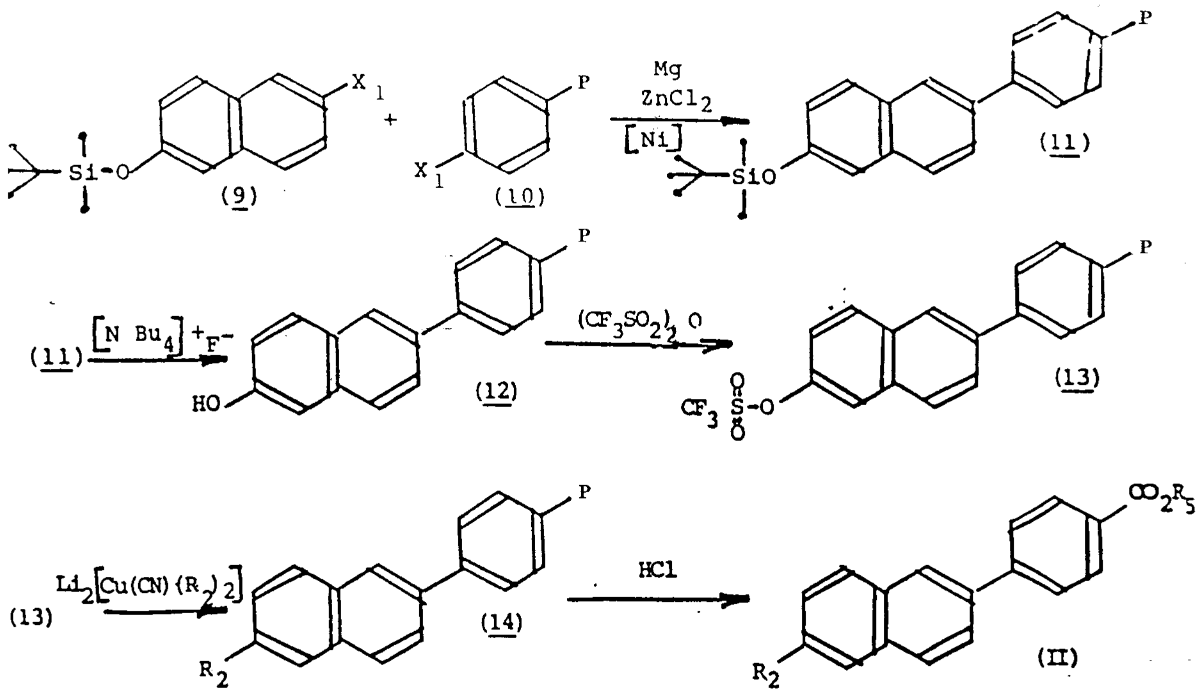


P = groupe protecteur de la fonction carbonyle
$X_1$ = Cl ou Br

Selon ce procédé on réalise une réaction de couplage entre un dérivé 6-halogéno 2-tert-butyl-diméthylsilyloxy naphtalène (9) et un dérivé carbonyle protégé d'un acide p-halogénobenzoïque (10). Selon ce procédé, le composé (10) est transformé en son magnésien, lithien ou zincique selon les méthodes connues dans la littérature et couplé avec le dérivé halogéné du naphtalène (9) en utilisant, comme catalyseur de réaction, un métal de transition ou l'un de ses complexes.

Comme catalyseurs on peut en particulier mentionner ceux dérivés du nickel ou du palladium et en particulier des composés du $Ni_{II}$ ($NiCl_2$) avec diverses phosphines en particulier le diphényl phosphino éthane.

La réaction de couplage est généralement effectuée à une température comprise entre -20 et +30 °C dans un solvant anhydre tel que par exemple le diméthylformamide ou le tétrahydrofuranne.

Divers groupes protecteurs peuvent être utilisés pour protéger la fonction carbonyle de l'acide p-halogénobenzoïque mais on préfère selon l'invention utiliser le groupe oxazolinyle.

Le dérivé benzonaphtalénique de formule (11) est ensuite traité par le fluorure de tétraméthylammonium dans le tétrahydrofuranne en vue d'obtenir le dérivé naphtol de formule (12). Ce dernier est alors transformé en dérivé trifluorométhanesulfonate de formule (13) qui, par traitement à l'aide d'un dérivé organocuprique approprié, conduit selon la méthode décrite par J.E Mc MURRY et al. (Tetrahédron Letters 24, p. 2723, 1983) au dérivé du naphtalène de formule (14) substitué en position 6 par un radical alkyle. Par élimination du groupe protecteur à l'aide d'acide chlorhydrique en solution aqueuse, on accède aux composés de formule (II) dans lesquels $R_5$ = H.

Pour obtenir les composés de formule (I) on peut utiliser également le procédé faisant intervenir les étapes représentées par le schéma réactionnel suivant:

SCHEMA D

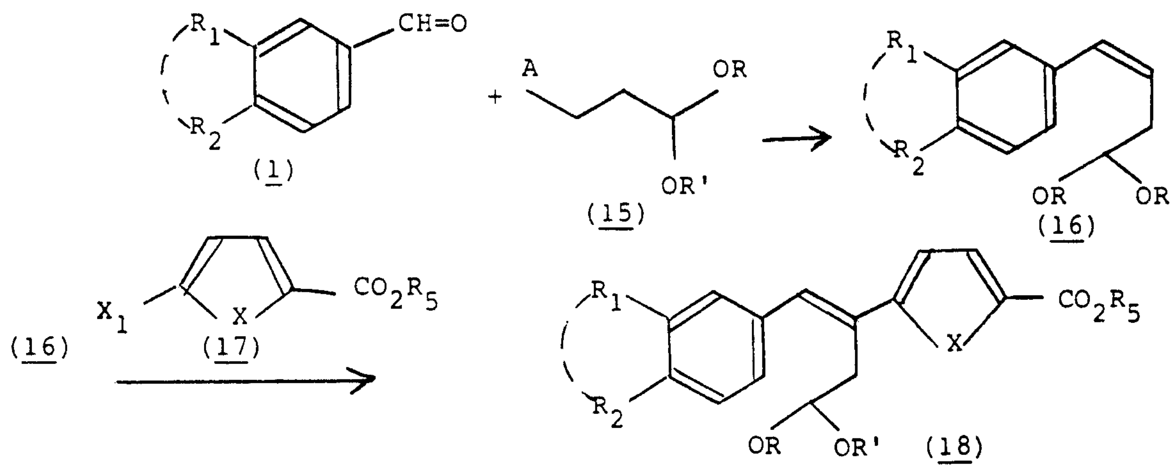

$X_1$ = Br ou Cl

Selon ce procédé on effectue une réaction de Wittig-Horner entre un aldéhyde aromatique (1) et un dérivé de phosphore pentavalent (15) dans lequel A a les mêmes significations que celles données pour le Schéma A ci-dessus, les conditions de réaction étant également les mêmes. On obtient ainsi le composé intermédiaire (16) qui est ensuite traité par un halogéno-ester hétérocylique (17) selon la réaction de HECK pour conduire au composé (18) qui est ensuite cyclisé.

Le couplage entre l'aldéhyde aromatique (1) et le dérivé de phosphore pentavalent (15) est réalisé de préférence par réaction de Wittig ($A = -P[\phi]^{\oplus}_3\ Br^{\ominus}$ ). On utilise de préférence comme base le tert-butylate de potassium dans le THF. Ceci permet l'obtention stéréospécifique du composé (16) sous sa forme Z. L'utilisation de bases lithiées telles que le di-isopropylamidure de lithium conduit à des mélanges E + Z de l'intermédiaire (16) . La réaction de Heck est effectuée de préférence entre 120 et 220 ° C (sous azote) et on opère généralement sans solvant. On peut utiliser comme bases des amines à haut point d'ébullition, par exemple du diaza bicycloundecene (DBU) mais les meilleurs résultats sont obtenus avec le carbonate de sodium ou de potassium finement broyé, le catalyseur utilisé étant l'acétate de palladium (II) en présence de triphénylphosphine.

A partir des esters et acides obtenus ci-dessus il est possible d'accéder selon les méthodes classiques aux composés de formule (I) dans lesquels $R_3$ a l'une quelconque des autres significations.

La présente invention a également pour objet les composés intermédiaires de synthèse représentés par la formule suivante:

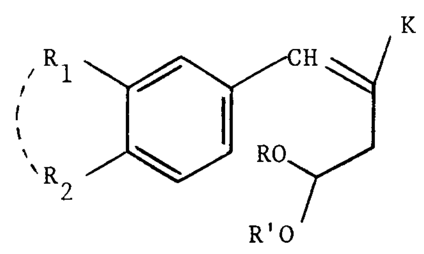

dans laquelle:

$R_1$ et $R_2$ ont les mêmes significations que données ci-dessus pour la formule (I),

K représentant un atome d'hydrogène ou le radical

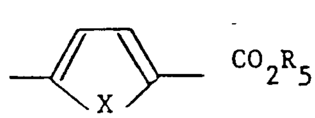

X et $R_5$ ayant les mêmes significations que ci-dessus pour la formule (I),

et R et R' représentent un radical alkyle inférieur ou pris ensemble forment un cycle dioxanne ou dioxolane.

La présente invention a également pour objet à titre de médicament les composés de formule (I) telle que définie ci-dessus.

Ces composés présentent une excellente activité dans le test d'inhibition de l'ornithine décarboxylase après induction, par "tape stripping", chez le rat nu. Ce test est admis comme mesure de l'action des rétinoïdes sur les phénomènes de prolifération cellulaire.

Ces composés conviennent particulièrement bien pour traiter les affections dermatologiques liées à un désordre de la kératinisation (différenciation-prolifération) ainsi que les affections dermatologiques, ou autres, à composante inflammatoire et/ou immunoallergique notamment :

- les acnés vulgaires, comédoniennes ou polymorphes, les acnés séniles, solaires, et les acnés médicamenteuses ou professionnelles
- les formes étendues et/ou sévères de psoriasis, et les autres troubles de la kératinisation, et notamment les ichtyoses et états ichtyosiformes
- la maladie de Darier
- les kératodermies palmo-plantaires
- les leucoplasies et états leucoplasiformes, le lichen plan
- toutes proliférations dermatologiques bénignes ou malignes, sévères ou étendues.

Ils sont également actifs dans le traitement des tumeurs, du psoriasis rhumatoïde, des atopies cutanées ou respiratoires ainsi que de certains problèmes ophtalmologiques relatifs aux cornéopathies.

La présente invention a donc également pour objet des compositions médicamenteuses contenant au moins un composé de formule (I) tel que défini ci-dessus, ou un de ses sels, à une concentration, de préférence entre 0,00001 et 5% en poids par rapport au poids total de la composition.

La présente invention a donc aussi pour objet une nouvelle composition médicamenteuse, destinée notamment au traitement des affections susmentionnées, caractérisée par le fait qu'elle comporte, dans un support pharmaceutique acceptable, au moins un composé de formule (I) et/ou un de ses sels.

Les composés selon l'invention présentent, par rapport aux rétinoïdes classiques, une meilleure stabilité à la lumière et à l'oxygène, ceci étant essentiellement dû au fait qu'ils ne possèdent pas de double liaison facilement isomérisable ou oxydable.

Les composés selon l'invention sont généralement administrés à une dose journalière d'environ 0,01 µg/kg à 0,1 mg/kg.

Comme support des compositions, on peut utiliser tout support conventionnel, le composé actif se trouvant soit à l'état dissous, soit à l'état dispersé dans le véhicule.

L'administration peut être effectuée par voie entérale, parentérale, topique ou oculaire. Par voie entérale, les médicaments peuvent se présenter sous forme de comprimés, de gélules, de dragées, de sirops, de suspensions, de solutions, de poudres, de granulés, d'émulsions. Par voie parentérale, les compositions peuvent se présenter sous forme de solutions ou suspensions pour perfusion ou pour injection.

Par voie topique, les compositions pharmaceutiques à base des composés selon l'invention se présentent sous forme d'onguents, de teintures, de crèmes, de pommades, de poudres, de timbres, de tampons imbibés, de solutions, de lotions, de gels, de sprays ou encore de suspensions.

Ces compositions par voie topique peuvent se présenter soit sous forme anhydre, soit sous forme aqueuse selon l'indication clinique.

Par voie oculaire, ce sont principalement des collyres.

Les composés de formule (I), selon l'invention, trouvent également une application dans le domaine cosmétique, en particulier dans l'hygiène corporelle et capillaire et notamment pour le traitement des peaux à tendance acnéique pour la repousse des cheveux, l'anti-chute, pour lutter contre l'aspect gras de la peau ou des cheveux, dans la protection contre les effets néfastes du soleil ou dans le traitement des peaux physiologiquement sèches.

La présente invention vise donc également une composition cosmétique contenant, dans un support cosmétiquement acceptable, au moins un composé de formule (I) ou un de ses sels, cette composition se présentant notamment sous forme de lotion, gel, savon ou shampooing.

La concentration en composé de formule (I), dans les compositions cosmétiques, est comprise entre 0,00001 et 2% en poids et de préférence entre 0,0001 et 1% en poids.

Les compositions médicamenteuses et cosmétiques selon l'invention peuvent contenir des additifs inertes ou même pharmacodynamiquement ou cosmétiquement actifs et notamment : des agents hydratants comme la thiamorpholinone et ses dérivés ou l'urée ; des agents antiséborrhéiques ou antiacnéiques, tels que la S-carboxyméthylcystéine, la S-benzyl-cystéamine,leurs sels et leurs dérivés, la tioxolone ou le

peroxyde de benzoyle ; des antibiotiques comme l'érythromycine et ses esters, la néomycine ou les tétracyclines ; des agents favorisant la repousse des cheveux, comme le "Minoxidil" (2,4-diamino-6-pipéridino-pyrimidine-3-oxyde) et ses dérivés, le Diazoxide (7-chloro-3-méthyl-1,2,4 benzothiadiazine-1,1-dioxyde) et le Phénytoïn (5,5-diphénylimidazolidine-2,4 dione) ; des agents anti-inflammatoires stéroïdiens et non stéroïdiens ; des caroténoïdes et, notamment, le $\beta$-carotène ; des agents anti-psoriasiques tels que l'anthraline et ses dérives et les acides eicosatétraynoïque-5,8,11,14 et triynoïque-5,8,11.

Les compositions selon l'invention peuvent également contenir des agents d'amélioration de la saveur, des agents conservateurs, des agents stabilisants, des agents régulateurs d'humidité, des agents régulateurs de pH, des agents modificateurs de pression osmotique des agents émulsionnants, des filtres UV-A et UV-B, des anti-oxydants tels de l'$\alpha$-tocophérol, le butylhydroxy-anisole ou le butylhydroxy toluène.

On va maintenant donner, à titre d'illustration et sans aucun caractère limitatif, plusieurs exemples de préparation des composés actifs de formule (I) selon l'invention ainsi que des exemples de compositions les contenant.

EXEMPLE I : Préparation de l'acide p-(6-tert-butyl-2-naphtyl) benzoïque

a) 6-bromo-2-tert-butyl diméthylsilyloxy naphtalène

On dissout dans 50ml de diméthylformamide :

- 10g (45 mmoles) de 6-bromo-2-naphtol
- 5,65g (55 mmoles) de triéthylamine
- 0,12g (1 mmole) de 4-diméthylaminopyridine

On ajoute lentement 7,55g (50 mmoles) de chlorure de tert-butyl diméthylsilyle et la solution résultante est agitée pendant trois heures à température ambiante. Le mélange est versé sur 200 ml d'eau puis acidifié avec de l'acide chlorhydrique concentré jusqu'à pH environ 1. La solution est extraite avec de l'éther (4 X 100ml). La solution éthérée est ensuite lavée avec de l'eau, séchée sur sulfate de sodium anhydre et le solvant évaporé.

On obtient ainsi une huile jaune pâle qui est purifiée, par élution avec de l'heptane, sur une courte colonne de silice. Après concentration du solvant, on obtient une huile incolore qui cristallise. Après séchage, on obtient 13,2g (Rendement 87%) du produit attendu : Point de fusion : 60-62 ° C.

b) 6-[p-(4,4-diméthyl-2-oxazolinyl)- phényl]-2-naphtol

On additionne 100 ml de THF anhydre à un mélange de 15,3g (60 mmoles) de p-(4,4-diméthyl-2-oxazolinyl)-bromobenzène, préparé selon la méthode de A. MEYERS & al, JOC, 39, 2787 (1974), et 1,75g (70 m.at-g) de magnésium. La réaction démarre spontanément. On chauffe ensuite le mélange réactionnel au reflux, pendant 3 heures. Après refroidissement, on ajoute 8,16g (60 mmoles) de chlorure de zinc anhydre. Le mélange est agité pendant 1 heure à température ambiante. A la suspension blanche obtenue on ajoute 9,5g (28 mmoles) du composé obtenu en (a) ci-dessus et 0,32g (0,6 mmole) de $NiCl_2$/diphénylphosphinoéthane. Le mélange est agité pendant 3 heures supplémentaires. On arrête la réaction par addition d'une solution aqueuse (2M) de chlorure d'ammonium. Le milieu réactionnel est ensuite extrait au dichlorométhane et la phase organique lavée avec de l'eau puis séchée sur sulfate de magnésium. Après évaporation du solvant on obtient une huile orange qui est purifiée par passage sur une courte colonne de silice, en utilisant comme éluant du dichlorométhane. Après concentration du solvant d'élution, on obtient, sous forme d'un solide blanc, le 2-tert-butyl diméthylsilyloxy 6-[p(-4,4-diméthyl-2-oxazolinyl)-phenyl]naphtalène. Ce solide est dissous dans 50ml de tétrahydrofuranne et traité par 35 ml d'une solution molaire de fluorure de tétrabutylammonium dans le tétrahydrofuranne. Le mélange réactionnel est agité à température ambiante pendant 3 heures, puis versé dans de l'eau et extrait au dichlorométhane. La phase organique, après avoir été lavée avec de l'eau, est séchée sur sulfate de magnésium anhydre et concentrée. On obtient une huile orange qui est purifiée par élution sur une courte colonne de silice, en utilisant comme éluant un mélange (99/1) de dichlorométhane et de méthanol. Les solvants d'élution sont évaporés et on obtient une huile jaune pâle qui cristallise. Après recristallisation dans le méthanol chaud, on obtient 5,5g de cristaux sous forme d'aiguilles jaune pâle (Rendement 64%) de point de fusion : 221-223 ° C.

c) Trifluorométhanesulfonate de 6-[p-(4,4-diméthyl-2-oxazolinyl)-phényl]-2-naphtyle

A une solution de 3,03g (30mmoles) de triéthylamine, 0,025g (0,2 mmole) de 4,4-diméthylaminopyridine

et de 5g (15,8 mmoles) du composé obtenu en (b) ci-dessus, dans 100 ml de dichlorométhane à 0°C, on ajoute 4,89g (17,4 mmoles) d'anhydride trifluorométhanesulfonique. Le mélange est agité à température ambiante pendant 2 heures. Le mélange réactionnel est versé dans de l'eau, puis on acidifie à pH 3 par addition d'acide chlorhydrique concentré.

On extrait au dichlorométhane et la phase organique, après avoir été lavée avec de l'eau, séchée sur sulfate de magnésium anhydre, et concentrée, conduit à un solide huileux. La purification est effectuée par élution sur une courte colonne de silice en utilisant un mélange (3/1) d'hexane/dichlorométhane comme éluant.

Après avoir évaporé sous pression réduite les solvants d'élution, on obtient un solide huileux que l'on recristallise par du cyclohexane chaud. On obtient 3g d'un solide cristallin blanc (Rendement 42%) de point de fusion : 134-135°C.

d) 2-[p-(6-tert-butyl-2-naphtyl)-phényl]-4,4 diméthyl oxazoline.

A un mélange de 0,9g (10 mmoles) de cyanure de cuivre dans le tétrahydrofuranne refroidi à -78°C, on ajoute goutte à goutte sous agitation, 8,7ml d'une solution hexanique, 2,3 Molaire (20 mmoles) de tert-butyl lithium. On laisse le mélange se réchauffer jusqu'à 0°C, puis on le refroidit à nouveau à -78°C pour additionner 1,5g (3,3 mmoles) du composé obtenu en (C) ci-dessus. On laisse ensuite le milieu réactionnel se réchauffer jusqu'à -20°C et on l'agite deux heures supplémentaires. On stoppe la réaction par addition d'une solution aqueuse molaire de chlorure d'ammonium. On extrait au dichlorométhane et la phase organique est ensuite lavée avec de l'eau, séchée sur sulfate de magnésium et concentrée sous pression réduite.

On obtient ainsi un solide jaune pâle qui est ensuite purifié par HPLC préparative (ZORBAX SIL, diisopropyl éther/iso-octane/triéthylamine 75/25/1). On obtient ainsi 0,35g (Rendement 30%) d'un solide jaune pâle de point de fusion :154-159°C.

e) acide p -(6-tert-butyl-2-naphtyl)-benzoïque.

0,3g (0.8 mmole) du composé obtenu en (d) ci-dessus est chauffé à reflux dans 20ml d'une solution d'acide chlorhydrique 3M, pendant 3 heures. On obtient un précipité blanc qui est filtré, redissous dans 20 cc de soude méthanolique à 20%. On chauffe le mélange au reflux pendant 30 mm. Le précipité blanc formé est filtré, lavé avec de l'eau et séché à 75°C sous vide pendant 24 heures.

On obtient ainsi 0,09g (Rendement 40%) de l'acide attendu de point de fusion : 310-315°C.

EXEMPLE II : Préparation de l'ester éthylique de l'acide p-(5,6,7,8-tétrahydro-5,5,8,8 - tétraméthyl-2-anthracényl)-benzoïque

a) Ester éthylique de l'acide p-(3,3-diméthoxypropyl)-benzoïque

On dissout 83,58g de p-bromobenzoate d'éthyle dans 100ml de dioxanne puis on ajoute successivement 3,83g de triphénylphosphine, 41,00g de diméthylacétal de l'acroléïne, 100 g de carbonate de potassium et 1,64g d'acétate de palladium (II).

Le mélange est chauffé à 110°C, en agitant pendant 16 heures. On filtre ensuite sur célite, lave la célite avec 3 x 200ml d'éther éthylique, sèche et évapore. On obtient ainsi 90,37g d'une huile jaune que l'on dissout dans 365 ml de méthanol. On ajoute 1,46g de palladium sur charbon (5%) et procède à l'hydrogènation. Quand l'absorption de l'hydrogène est terminée, le catalyseur est éliminé par filtration sur célite. Après évaporation du méthanol, le résidu est chromatographié (colonne de silice 30 x 10cm, éluant : mélange de dichlorométhane 50% et d'hexane 50%).

On obtient ainsi, après évaporation des solvants, 72,77g (Rendement 79%) d'ester éthylique de l'acide p-(3,3-diméthoxypropyl)-benzoïque.

b) Ester éthylique de l'acide p-(1-diéthoxyphosphoryl-3,3-diméthoxypropyl)-benzoïque

On dissout 70,00g de produit obtenu en (a) ci-dessus dans 1000ml de tétrachlorure de carbone. On ajoute 1g de peroxyde de benzoyle puis 49,38g de N-bromo succinimide, par fractions. On chauffe ensuite à reflux pendant 45mn. On élimine le succinimide formé par filtration sur célite puis on évapore le solvant. On sèche ensuite l'huile obtenue à température ambiante, sous vide (1mm Hg).

L'huile est dissoute dans 400ml de triéthylphosphite, et on chauffe à 160°C pendant 18 heures. On

évapore le triéthylphosphite (120 °C, sous vide de la trompe à eau). Le résidu est déposé sur une colonne de silice (30 x 10cm) puis élué par un mélange d'acétate d'éthyle (70%) et d'hexane (30%).

On obtient ainsi 46,64g (Rendement 45%) d'ester éthylique de l'acide p-(1-diéthoxyphosphoryl-3,3-diméthoxypropyl)-benzoïque.

c) Ester éthylique de l'acide p-[1-(2,2-diméthoxyéthyl)-2-(5,6,7,8-tétrahydro-5,5,8,8-tétraméthyl-2-naphtyl)-vinyl]benzoïque

31,76g du phosphonate obtenu ci-dessus en (b) sont dissous dans 75ml de tétrahydrofuranne. On refroidit cette solution à -78 °C et on ajoute goutte à goutte une solution de diisopropylamidure de lithium, préparée à partir de diisopropylamine (12,5ml) et de n butyl lithium (1,6 m dans l'hexane) dans 75 ml de tétrahydrofuranne.

A cette solution rouge ainsi obtenue, on ajoute une solution de 5,6,7,8-tétrahydro-5,5,8,8-tétraméthyl-2-naphtaldéhyde (16,08g) dans 50ml de tétrahydrofuranne. On agite. 90mn à -78 °C puis 90mn à température ambiante.

On ajoute ensuite 150ml d'eau et extrait à l'éther (3 x 200ml).

La phase organique est lavée avec une solution saturée de chlorure de sodium, séchée (sulfate de magnésium). On filtre et évapore les solvants. Le résidu est chromatographié sur une colonne de silice (30 x 10cm) en utilisant comme éluant un mélange d'éther éthylique (30%) et d'hexane (70%).

On obtient ainsi successivement :

A) - 8,02g de p-[(E)-[1-(2,2-diméthoxyéthyl)-2-(5,6,7,8-tétrahydro- 5,5,8,8-tétraméthyl-2-naphtyl)]-vinyl]-benzoate d'éthyle de point de fusion : 82 °C.

B) - 8,24g de p-[(Z)-[1-(2,2-diméthoxyéthyl)-2-(5,6,7,8-tétrahydro-5,5,8,8-tétraméthyl-2-naphtyl)]-vinyl]-benzoate d'éthyle.

Le rendement en A + B est de 50%.

d) Ester éthylique de l'acide p-(5,6,7,8-tétrahydro-5,5,8,8-tétraméthyl-2-anthracényl)-benzoïque.

i) METHODE 1

7,02g de l'isomère (E) de l'ester obtenu en (c) ci-dessus sont dissous dans 80ml de dichlorométhane. On ajoute 2ml d'ester triméthylsilylique de l'acide trifluorométhane sulfonique et on agite 15 mn à température ambiante. On évapore le dichlorométhane et on applique le résidu au sommet d'une courte colonne de silice (5 x 10cm). On élue avec un mélange de chlorure de méthylène (60%) et d'heptane (40%). Par évaporation des solvants, on obtient 5,65g (Rendement 95%) d'ester éthylique de l'acide p-(5,6,7,8-tétrahydro-5,5,8,8-tétraméthyl-2-anthracényl)-benzoïque de point de fusion : 126 °C.

ii) METHODE II

7,80g de l'isomère (Z) de l'ester obtenu en (c) ci-dessus sont dissous dans 350ml de dichlorométhane. On ajoute 1ml d'ester triméthylsilylique de l'acide trifluorométhanesulfonique et on place le mélange réactionnel dans un réacteur photochimique. On agite pendant 3 heures, à température ambiante, en irradiant (lampe Hanovia moyenne pression, sans filtre). Le dichlorométhane est évaporé et le résidu vert foncé est déposé au sommet d'une colonne de silice (30 x 8cm). On élue avec un mélange de dichlorométhane (40%) et d'heptane (60%). On obtient ainsi, après évaporation des solvants, 2,67g (Rendement 40%) d'ester éthylique de l'acide p-(5,6,7,8-tétrahydro-5,5,8,8-tétraméthyl-2-anthracényl)-benzoïque.

EXEMPLE III

Acide p-(5,6,7,8-tétrahydro-5,5,8,8-tétraméthyl-2-anthracényl)-benzoïque

7,32g de l'ester obtenu à l'exemple II (d) sont mis en suspension dans 400ml d'éthanol. On ajoute 38ml de soude 5 N et on chauffe à 60 °C pendant 60 minutes. On évapore l'éthanol, reprend par de l'eau (500ml) et acidifie jusqu'à pH 1 (avec HCl 6N). On extrait à l'éther (3 x 500 ml) et la phase organique est séchée ($MgSO_4$). Après filtration et évaporation des solvants, on ajoute au résidu 30 ml d'éther éthylique. On obtient ainsi, après agitation et filtration, 6,25g (Rendement 92%) d'acide p-(5,6,7,8-tétrahydro-5,5,8,8-tétraméthyl-2-anthracényl)-benzoïque de point de fusion : 282 °C.

EXEMPLE IV

Ester méthylique de l'acide p-[7-(1-adamantyl)-6-méthoxy-2-naphtyl]-benzoïque

a) 3-(1-adamantyl)-4 méthoxy benzaldéhyde.

11,90g (37mmoles) de 2-(1-adamantyl)-4-bromo anisole dissous dans 85ml de THF sont ajoutés goutte à goutte à 1g de magnésium et un cristal d'iode. Au début de l'addition, on chauffe le milieu réactionnel jusqu'à ce que la réaction soit initiée, puis le reste de la solution est ajouté de manière à maintenir un reflux régulier. On chauffe à reflux 30 minutes après la fin de l'addition puis ajoute 2,70g (37 mmoles) de DMF sec. On agite 30mn sans chauffer puis verse dans un mélange d'acide chlorhydrique aqueux (2N) et de dichlorométhane (300ml).

La phase organique est décantée et la phase aqueuse extraite au dichlorométhane. Les extraits organiques sont réunis, lavés avec une solution saturée de bicarbonate de sodium, puis une solution saturée de chlorure de sodium. On sèche sur sulfate de magnésium filtre et évapore les solvants.

Le résidu ainsi obtenu est purifié par passage sur colonne de silice (éluant: mélange de dichlorométhane (60%) et d'hexane (40%). Après évaporation des solvants on obtient l'aldéhyde attendu: 8,0g (80%) sous la forme d'une poudre jaune clair qui fond à 180°C.

b) Ester méthylique de l'acide p-[1-(2,2-diméthoxyéthyl)-2-[3-(1-adamantyl)-4-méthoxyphényl]-vinyl]-benzoïque.

11,42g (30,51 mmoles) du phosphonate obtenu à l'exemple II (b) sont dissous dans 50ml de THF. On refroidit à -78°C et ajoute goutte à goutte une solution de di-isopropylamidure de lithium (préparé de la manière habituelle à partir de 3,4g (33,58 mmoles) de di-isopropylamine et de 2,1ml (33,56 mmoles) d'une solution de n-butyllithium (1,6M dans l'hexane). La solution rouge-orange ainsi obtenue est agitée 30mn à -78°C puis on ajoute par portions une suspension de 3-(1-admantyl)-4-méthoxybenzaldéhyde obtenu ci-dessus en (a), (7,5g; 27,74 mmoles) dans 140ml de THF. Cette addition dure environ 10mn. On agite le mélange réactionnel 20mn à -78°C puis 2h à 25°C, verse dans l'eau, extrait trois fois par 100ml d'éther éthylique, lave la phase organique avec une solution saturée de chlorure de sodium, sèche sur sulfate de magnésium, puis filtre et évapore les solvants. Le résidu est chromatographié sur colonne de silice comme à l'exemple II (c). On recueille ainsi le mélange des deux isomères E et Z de l'ester méthylique de l'acide p-[1-(2,2-diméthoxyéthyl)-2-[3-(1-adamantyl)-4-méthoxyphényl]-vinyl]-benzoïque (7,5g, 53%) (huile jaune clair cristallisant partiellement). Ce mélange est utilisé tel quel pour la suite de la synthèse.

c) Ester méthylique de l'acide p-[7-(1-adamantyl)-6-méthoxy-2-naphtyl]-benzoïque.

6,65g (13,55 mmole) du mélange obtenu ci-dessus en (b) dans 400ml de dichlorométhane. On ajoute 2ml de l'ester triméthylsilylique de l'acide trifluorométhane sulfonique (TMSOTf) et irradie aux ultra-violets (UV) comme en II (d), pendant 4h. Les solvants sont évaporés, puis le mélange réactionnel est purifié par passage sur une colonne de silice (éluant: mélange de di-chlorométhane (70%) et d'hexane (30%). Les fractions contenant le produit attendu sont concentrées sous vide. Le résidu obtenu est filtré, lavé par 300ml d'hexane froid, puis séché sous vide à température ambiante. On obtient ainsi 4,65g (80%) du produit attendu, sous la forme d'un solide blanc jaune, qui fond à 245°C.

EXEMPLE V

Acide p-[7-(1-adamantyl)-6-méthoxy-2-naphtyl]-benzoïque

L'ester obtenu en IV (c) 1,28g, 3 mmoles) est mis en suspension dans 60ml de méthanol. On ajoute 6ml de soude 5N et agite tout en chauffant à reflux pendant 6h. On ajoute ensuite 200ml de méthanol et 200ml d'eau. La solution ainsi obtenue est concentrée de manière à éliminer la plus grande partie de méthanol, puis on ajoute 300ml d'éther éthylique et 200ml d'acide chlorhydrique 2N. L'acide brut précipite. On élimine la phase aqueuse, ajoute du THF (300ml), sèche sur sulfate de magnésium et évapore. Le solide obtenu est repris par 300ml d'hexane, filtré et séché à l'étuve à 100°C sous vide, pendant 16h. On obtient ainsi 1,16g (94%) d'acide attendu sous la forme d'une poudre blanc-gris qui fond à 360°C.

EXEMPLE VI

Ester méthylique de l'acide p-(7-tert-butyl-6-méthoxy-2-naphtyl)-benzoïque

a) Ester méthylique de l'acide p-[1-(2,2-diméthoxyéthyl)-2-(3-tert butyl-4-méthoxyphényl)-vinyl]-benzoïque.

5,49g (28,56 mmoles) d'aldéhyde 3-tert-butyl-4-méthoxy-benzoïque sont dissous dans 100ml de THF. On refroidit à -78°C et ajoute ensuite une solution de diisopropylamidure de lithium (34,55 mmoles) dans le THF (50ml), préparée comme ci-dessus. La réaction est effectuée comme en IV (c). Après extraction trois fois par 200ml d'éther et traitement comme en IV (b), on obtient 5,73g (49%) du mélange des esters E et Z attendus sous forme d'une huile jaune.

b) Ester méthylique de l'acide p-(7-tert-butyl-6-méthoxy-2-naphtyl)-benzoïque.

On dissout 5,67g (13,74 mmoles) du mélange d'esters obtenu en VI (a) dans 400ml de dichlorométhane. On ajoute 1ml de TMSOTf et irradie aux UV pendant 2h. Le solvant est évaporé et le résidu est chromatographié sur colonne de silice (éluant: mélange $CH_2Cl_2$ 60%, hexane 40%). On obtient ainsi 3,00g (63%) d'ester méthylique de l'acide p-(7-tert-butyl-6-méthoxy-2-naphtyl)-benzoïque qui fond à 133°C.

## EXEMPLE VII

Acide p-(7-tert-butyl-6-méthoxy-2-naphtyl)-benzoïque

L'ester obtenu en VI (b) est dissous dans 60ml de méthanol. On ajoute 6ml de soude 5N et chauffe à reflux pendant 1h. Le méthanol est ensuite évaporé et on ajoute 300ml d'eau. On extrait par cinq fois 100ml d'éther, sèche (Mg $SO_4$), filtre et évapore sous pression réduite. Le solide obtenu est repris par 100ml d'hexane, filtré et séché. On obtient ainsi 0,92g (96%) de l'acide attendu, qui fond à 283°C.

## EXEMPLE VIII

Ester méthylique de l'acide p-[7-(1-adamantyl)-6-hydroxy-2-naphtyl]-benzoïque

a) 2-[3-[3-(1-adamantyl-)-4-tert-butyl-diméthylsilyloxyphényl]-allyl]-1,3 dioxanne.

33g (72,1 mmoles de bromure de [2-(1,3-dioxan-2-yl) éthyl] triphénylphosphonium sont mis en suspension dans 100ml de THF. On refroidit à 0°C et ajoute par petite quantités 8,5g (75,6 mmoles) de terbutylate de potassium. On laisse remonter à 20°C et agite pendant 1h. On refroidit à 0°C et ajoute lentement une solution de 3-(1-adamantyl)-4-tert-butyl-diméthylsilyloxybenzaldéhyde dans 100ml de THF. Une fois l'addition terminée, on laisse revenir à température ambiante et agite pendant 2h. On verse dans l'eau, extrait au chlorure de méthylène, décante la phase organique, lave à l'eau, sèche ($MgSO_4$), et évapore les solvants. Le résidu est purifié par passage sur colonne de silice (éluant: mélange $CH_2Cl_2$ (50%), hexane (50%)). On obtient ainsi 19,4g (95%) de mélange attendu contenant plus de 90% d'isomère Z, et moins de 10% d'isomère E.

b) Ester méthylique de l'acide p-[1-[(1,3-dioxan-2-yl)méthyl]-2-[3-(1-adamantyl)-4-tert-butyl-diméthylsilyloxyphényl]-vinyl]-benzoïque.

On ajoute successivement dans un ballon:

19,1g (40,8 mmoles) du dérivé de dioxanne préparé en VIII (a), 8,8g de p-bromobenzoate de méthyle, 183mg (0,8 mmole) d'acétate de palladium, 428mg (1,6 mmole) de triphénylphosphine et 11,3g (81,6 mmoles) de carbonate de potassium finement broyé. Ce mélange est chauffé sous azote à 180°C pendant 2h. Après refroidissement à température ambiante, le solide obtenu est traité par un mélange de dichlorométhane et d'eau. La phase organique est décantée, lavée à l'eau, séchée ($MgSO_4$), filtrée et évaporée. Le résidu est chromatographié sur colonne de silice (éluant: $CH_2Cl_2$: 80%, hexane: 20%) pour donner 9,5g (39%) de l'ester méthylique de l'acide p-[1-[(1,3-dioxan-2-yl)-méthyl]-2-[3-(1-adamantyl)-4-tert-butyl-diméthylsilyloxyphényl]-vinyl]-benzoïque.

c) Ester méthylique de l'acide p-[7-(1-adamantyl)-6-tert-butyl-diméthylsiloxy-2-naphtyl]-benzoïque.

9,0g (14,9 mmoles) d'ester obtenu en VIII (b) sont dissous dans 400ml de dichlorométhane. On ajoute

2ml de TMSOTf et agite pendant 3h30 sous azote et sous irradiation UV. Après évaporation du dichlorométhane, le résidu est déposé sur colonne de silice et élué avec un mélange de dichlorométhane (50%) et d'hexane (50%); après évaporation des solvants, le solide jaune clair obtenu est agité dans 100ml d'hexane froid; par filtration on obtient 4,55g (58%) de l'ester attendu, sous forme d'une poudre blanche fondant à 185 ° C.

d) Ester méthylique de l'acide p-[7-(1-adamantyl)-6-hydroxy-2-naphtyl]-benzoïque.

4,55g (8,6 mmoles) de l'ester obtenu en VIII (c) sont dissous dans 30ml de THF sec. On ajoute goutte à goutte 9,5ml d'une solution M de fluorure de tétrabutylammonium dans le THF. La solution se colore en orange. On agite 2,5h à 20 ° C, évapore le THF, ajoute de l'eau et extrait trois fois par 200ml d'éther éthylique. La phase organique est lavée avec une solution saturée de chlorure de sodium, séchée (MgSOu), puis évaporée. On obtient ainsi 3,51g (99%) de l'ester attendu sous la forme d'un solide blanc à beige qui fond à 247 ° C.

EXEMPLE IX

Acide p-[7-(1-adamantyl)-6-hydroxy-2-naphtyl]-benzoïque

L'ester obtenu en VIII (c) (1,24g, 3 mmoles) est mis en suspension dans 60ml de méthanol. On ajoute 6ml de soude 5N et chauffe à reflux pendant 1h. Le méthanol est évaporé, on ajoute 200ml d'acide chlorhydrique 2N et extrait 3 fois par 400ml d'éther éthylique. La phase organique est lavée par une solution saturée de bicarbonate de sodium, puis de NaCl, séchée et évaporée. On obtient ainsi 1,08g (90%) de l'acide attendu, qui fond à 295-300 ° C.

EXEMPLE X

Ester méthylique de l'acide 5-(5,6,7,8-tétrahydro-5,5,8,8-tétraméthyl-2-anthracényl)-2-furannecarboxylique

a) 2-[3-(5,6,7,8-tétrahydro-5,5,8,8-tétraméthyl-2-naphtyl)-allyl]-1,3-dioxanne.

De manière analogue à l'exemple VIII (a), à partir de 25,2g (55 mmoles) de bromure de [2-(1,3-dioxan-2-yl) éthyl] triphénylphosphonium et de 10,8g (50 mmoles) de 5,6,7,8-tétrahydro-5,5,8,8-tétraméthyl-2-naphtaldéhyde, on obtient après chromatographie sur colonne de silice (éluant: hexane 95%, éther 5%), 4g (66%) de 2-[3-(5,6,7,8-tétrahydro-5,5,8,8-tétraméthyl-2-naphtyl)-allyl]-1,3-dioxanne sous la forme d'une huile jaune.

b) Ester méthylique de l'acide E-5-[1-[(1,3-dioxan-2-yl) méthyl]-2-(5,6,7,8-tétrahydro-5,5,8,8-tétraméthyl-2-naphtyl)-vinyl]-2-furanne-carboxylique.

9,42g (30 mmoles) du dérivé de dioxanne substituées obtenu ci-dessus en (a), 6,15g (30 mmoles) de 5-bromo-2-furoate de méthyle, 135mg (0,6 mmole) de Pd $(OAc)_2$, 315mg (1,2 mmole) de $P(C_6H_5)_3$ et 8,2g (60 mmoles) de carbonate de potassium finement broyé sont chauffés à 160 ° C pendant 2h. On ajoute alors 3,07g (15 mmoles) de bromofuroate de méthyle, 135mg de Pd $(OAc)_2$ et 315mg de $P(C_6H_5)_3$. Le chauffage est poursuivi pendant encore 2h. Le mélange réactionnel est alors refroidi et purifié par passage sur colonne de silice (éluant: dichlorométhane 80%, hexane 20%). On obtient ainsi 7,1g (53%) de l'ester attendu qui fond à 123-124 ° C.

c) Ester méthylique de l'acide 5-(5,6,7,8-tétrahydro-5,5,8,8-tètraméthyl-2-anthracényl)-2-furannecarboxylique.

7g de l'ester obtenu en X (b) (16 mmoles) sont dissous dans 200ml de dichlorométhane. On refroidit à 0 ° C et ajoute 1ml de TMSOTf. On laisse revenir à température ambiante et agite 30 mn. Le solvant est évaporé et le résidu purifié par passage sur colonne de silice (éluant: dichlorométhane 50%, hexane 50%). Le produit ainsi obtenu peut être recristallisé dans l'hexane pour donner 5,5g (96%) de l'ester attendu qui fond à 127 ° C.

EXEMPLE XI

Acide 5-(5,6,7,8-tétrahydro-5,5,8,8-tétramethyl-2-anthracényl)-2-furannecarboxylique

5,2g (14 mmoles) de l'ester obtenu en X (c) sont traités 4h au reflux par 200ml de soude méthanolique 2N. On évapore, reprend par l'eau, acidifie (HCl concentré) extrait à l'éther, lave à l'eau, sèche ($MgSO_4$), évapore et recristallise dans un mélange d'éther isopropylique et d'acétate d'éthyle (50%). On obtient ainsi 4,6g (93%) d'acide 5-(5,6,7,8-tétrahydro-5,5,8,8-tétraméthyl-2-anthracényl)-2-furannecarboxylique qui fond à 229-230 ° C.

EXEMPLE XII

Ester méthylique de l'acide 5-(5,6,7,8-tétrahydro-5,5,8,8-tétraméthyl-2-anthracényl)-2-thiophènecarboxylique

a) Ester méthylique de l'acide 5-[1-[(1,3-dioxan-2-yl) méthyl]-2-(5,6,7,8-tétrahydro-5,5,8,8-tétraméthyl-2-naphtyl)-vinyl]-2-thiophène carboxylique.

Dans un ballon on place: 9,42g (30 mmoles) du dérivé de dioxanne obtenu en X (a), 6,63g (30 mmoles) de 5-bromo-2-thiophènecarboxylate de méthyle, 135mg de Pd $(OAc)_2$, 315mg (1,2 mmole) de $P(C_6H_5)_3$, 8,3g (60 mmoles) de $K_2CO_3$ finement broyé. Le mélange est chauffe sous azote à 200 ° C pendant 2h. On ajoute alors 3,3g (15 mmoles) de 5-bromothiophènecarboxylate de méthyle et chauffe encore pendant 2h.

On purifie grossièrement sur colonne de silice (éluant: dichlorométhane 80%, hexane 20%) pour obtenir 5,4g d'un mélange non séparable dans les conditions utilisées, du produit attendu (60%) et de E-2-[3-(5,6,7,8-tétrahydro-5,5,8,8-tétraméthyl)-2-naphtyl] -allyl-1,3-dioxanne (40%). Ce mélange est utilisé tel quel pour la suite de la synthèse.

b) Ester méthylique de l'acide 5-(5,6,7,8-tétrahydro-5,5,8,8-tétraméthyl-2-anthracényl)-2-thiophènecarboxylique

Le mélange obtenu en XII (a) (5,2g) est dissous dans 100ml de dichlorométhane. On refroidit à 0 ° C et ajoute 500µl de TMSOTf. On laisse revenir à 20 ° C et agite pendant 30 mn. On évapore le solvant et purifie le résidu par passage sur colonne de silice (éluant: $CH_2Cl_2$ (50%), hexane (50%)). Le produit ainsi obtenu peut être recristallisé dans le cyclohexane pour donner 2,5g d'ester méthylique de l'acide 5-(5,6,7,8-tétrahydro-5,5,8,8-tétraméthyl-2-anthracényl)-2-thiophène carboxylique, qui fond à 147-148 ° C.

EXEMPLE XIII

Acide 5-(5,6,7,8-tétrahydro-5,5,8,8-tétraméthyl-2-anthracényl)-2-thiophène carboxylique

2,1g (5,5 mmoles) d'ester obtenu en XII (b) sont traités au reflux pendant 8h par 100ml de soude méthanolique 2N. On évapore le méthanol, reprend par l'eau, acidifie (HCl concentré), extrait à l'éther, sèche ($MgSO_4$) et évapore. Le résidu est recristallisé dans un mélange d'éther isopropylique (60%) et d'acétate d'éthyle (40%). On obtient ainsi 5g (90%) d'acide 5-(5,6,7,8-tétrahydro-5,5,8,8-tétraméthyl-2-anthracényl)-2-thiophènecarboxylique, qui fond à 254-255 ° C.

EXEMPLE XIV

Ester méthylique de l'acide p-[3,4(2H)-dihydro-4,4-diméthyl-7-naphto-[2,3-b]pyrannyl]-benzoïque

a) Ester méthylique de l'acide p-[1-(2,2-diméthoxyéthyl)-2-(4,4-diméthyl-6-chromanyl)-vinyl]benzoïque.

De manière analogue à l'exemple IV, 6,49g (17,3 mmoles)de phosphonate préparé à l'exemple II (b) dissous dans le THF (20ml) sont traités par une solution de di-isopropylamidure de lithium (17,3 mmoles) dans le THF (20ml). On ajoute une solution de 6-formylchromane et agite pendant 2h à -78 ° C. On laisse revenir à température ambiante, verse dans l'eau et extrait à l'éther. La phase organique est séchée ($MgSO_4$), les solvants sont évaporés et le résidu obtenu est chromatographié sur colonne de silice (éluant: hexane). On obtient ainsi 950mg (15%) du mélange E,Z des esters méthyliques de l'acide p-[[1-(2,2-diméthoxyéthyl)-2-(4,4-diméthyl-6-chromanyl)]-vinyl]-benzoïque sous forme d'une huile jaune pâle qui est utilisée telle quelle pour la suite de la synthèse.

b) Ester méthylique de l'acide p-[3,4(2H)-dihydro-4,4-diméthyl-7-naphto[2,3-b]pyrannyl]-benzoïque.

950g (2,3 mmoles) du mélange E + Z d'esters obtenus ci-dessus en (a) sont dissous dans 200ml de dichlorométhane. On ajoute 0,5ml de TMSOTf et irradie aux UV, sous azote, en agitant. Au bout de 3h, les solvants sont évaporés. Le résidu est chromatographié sur colonne de silice (4x25cm, éluant: mélange dichlorométhane 50%, hexane 50%). Par évaporation on obtient 430mg (54%) d'ester méthylique de l'acide p-[3,4(2H)-dihydro-4,4-diméthyl-7-naphto [2,3-b]pyrannyl]-benzoïque, qui fond à 153-154 ° C.

EXEMPLE XV

Acide p-[3,4(2H)-dihydro-4,4-diméthyl-7-naphto[2,3-b]pyrannyl]-benzoïque

370mg (1,06 mmole) d'ester obtenu à l'exemple XIV (b) sont mis en suspension dans 40ml de méthanol. On ajoute 400mg de soude en pastilles et chauffe à reflux pendant 2h en agitant. On évapore le méthanol, rajoute 300ml d'eau et neutralise (HCl N). On extrait à l'éther (3x300ml), lave la phase organique à l'eau saturée en NaCl, sèche ($MgSO_4$) et évapore les solvants. On obtient ainsi 240mg (68%) d'acide p-[3,4(2H)-dihydro-4,4-diméthyl-7-naphto [2,3-b]pyrannyl]-benzoïque qui fond à 249-250 ° C.

EXEMPLE XVI

Ethylamide de l'acide 5-(5,6,7,8-tétrahydro-5,5,8,8-tétraméthyl-2-anthracényl)-2-furannecarboxylique

a) Chlorure de l'acide 5-(5,6,7,8-tétrahydro-5,5,8,8-tétraméthyl-2-anthracényl)-2-furannecarboxylique.

1,15g (3,31 mmoles) d'acide obtenu en XI sont traités par 5ml de chlorure de thionyle à 40 ° C pendant 1h. On évapore à sec, reprend par du benzène et réévapore. On obtient ainsi 1,17g d une masse cristalline foncée que l'on utilise telle quelle pour la suite de la synthèse.

b) Ethylamide de l'acide 5-(5,6,7,8-tétrahydro-5,5,8,8-tétraméthyl-2-anthracényl)-2-furannecarboxylique.

200mg (0,54 mmole) du chlorure d'acide obtenu ci-dessus en (a) dissous dans 20ml de $CH_2Cl_2$ sec sont ajoutés à une solution d'éthylamine (184mg; 4,08 mmoles) dans 1ml de $CH_2Cl_2$. On agite à température ambiante 15mn, ajoute de l'eau, acidifie à pH 1 (HCl N), extrait ($CH_2Cl_2$), lave avec une solution saturée de $NaHCO_3$, puis de l'eau, sèche, et évapore les solvants. On obtient ainsi 110mg (54%) de l'amide attendu qui fond à 200 ° C.

EXEMPLE XVII

Morpholide de l'acide 5-(5,6,7,8-tétrahydro-5,5,8,8-tétraméthyl-2-anthracényl)-2-furannecarboxylique

A 200mg (0,54 mmole) du chlorure d'acide obtenu en XVI (a) dissous ans 20ml de $CH_2Cl_2$ sont ajoutés 237mg (2,72 mmoles) de morpholine. On agite pendant 15mn, ajoute de l'eau, acidifie à pH 1 (HCl N) et recueille la phase organique. On lave avec une solution saturée de $NaHCO_3$, de l'eau, sèche ($MgSO_4$) et évapore. Le résidu obtenu est purifié par passage sur une colonne de silice (éluant: mélange $CH_2Cl_2$ (95%) et acétone (5%)). Par évaporation des solvants on obtient 193mg (85%) du morpholide de l'acide 5-(5,6,7,8-tétrahydro-5,5,8,8-tétraméthyl-2-anthracényl)-2-furannecarboxylique qui fond à 161 ° C.

EXEMPLE XVIII

5-(5,6,7,8-tétrahydro-5,5,8,8-tétraméthyl-2-anthracényl)-2-furannecarboxylate de 2-hydroxyéthyle

On dissout 340mg (5,4 mmoles) d'éthylèneglycol dans 1ml de $CH_2Cl_2$. On ajoute 86mg (1,1 mmole) de pyridine et 200mg (0,54 mmole) de chlorure d'acide obtenu en XVI (a) dissous dans 20ml de $CH_2Cl_2$. On agite à température ambiante pendant 15mn. On ajoute de l'eau, acidifie à pH 1 (HCl N), extrait au dichlorométhane, lave successivement avec une solution saturée de bicarbonate de sodium, puis de l'eau; sèche ($MgSO_4$), filtre, évapore et passe le résidu obtenu sur colonne de silice (éluant: $CH_2Cl_2$ 95%, acétone 5%). Par évaporation du solvant on obtient 152mg (71%) de l'ester attendu qui fond à 143 ° C.

EXEMPLE XIX

Alcool p-(5,6,7,8-tétrahydro-5,5,8,8-tétraméthyl-2-anthracényl)-benzylique

300mg (0,83 mmole) d'acide p-(5,6,7,8-tétrahydro-5,5,8,8-tétraméthyl-2-anthracényl)-benzoïque obtenu en III sont dissous dans 25ml de THF. On ajoute 48mg (1,25 moles) de Li Al $H_4$, agite 15mn à température ambiante puis 10mn à reflux. On laisse revenir à température ambiante, puis ajoute 28µl d'une solution saturée de tartrate de sodium et potassium. On filtre, évapore le THF, reprend dans l'hexane: le précipité formé est filtré pour donner 167mg (57%) de l'alcool attendu qui fond à 131 ° C.

EXEMPLES DE FORMULATION

A - VOIE ORALE

| 1) Comprimé de 0,2g | |
|---|---|
| Acide p-(5,6,7,8- tétrahydro-5,5,8,8 -tétraméthyl-2-anthracényl) -benzoïque | 1 µg |
| Amidon | 0,114g |
| Phosphate bicalcique | 0,020g |
| Silice | 0,020g |
| Lactose | 0,030g |
| Talc | 0,010g |
| Stéarate de magnésium | 0,005g |

Dans cet exemple le composé actif peut être remplacé par la même quantite' de l'ester méthylique de l'acide 5-(5,6,7,8-tétrahydro-5,5,8,8'-tétraméthyl-2-anthracényl)-2-thiophènecarboxylique.

| 2) Capsule de 0,4g contenant une suspension | |
|---|---|
| Acide p-(6-tert-butyl-2-naphtyl) benzoïque | 0,001g |
| Glycérine | 0,200g |
| Saccharose | 0,050g |
| Polyéthylèneglycol 400 | 0,050g |
| Eau purifiée Q.S.P. | 0,400g |

Cette suspension est conditionnée dans une capsule composée de gélatine, glycérine, dioxyde de titane et eau.

Dans cet exemple le composé actif peut être remplacé par la même quantité de l'ester méthylique de l'acide p-(7-tert-butyl-6-méthoxy-2-naphtyl)-benzoïque.

B - VOIE TOPIQUE

| 1) Onguent | |
|---|---|
| Acide p-(5,6,7,8- tétrahydro-5,5,8,8 -tétraméthyl-2-anthracényl) -benzoïque | 0,0001g |
| Alcool stéarylique | 3,000g |
| Lanoline | 5,000g |
| Vaseline | 15,000g |
| Eau distillée Q.S.P. | 100,000g |

Dans cet exemple le composé actif peut être remplacé par la même quantité de l'ester éthylique de l'acide p-(5,6,7,8-tétrahydro-5,5,8,8-tétraméthyl-2-anthracényl)-benzoïque.

| 2) Gel | |
|---|---|
| Ester éthylique de l'acide p-(5,6,7,8-tétrahydro -5,5,8,8-tétraméthyl-2-anthracényl) -benzoïque | 0,005g |
| Hydroxy -propyl cellulose vendue par la société Herculès sous le nom de "KLUCEL HF" | 2,000g |
| Eau/éthanol (50:50) Q.S.P. | 100,000g |

Dans cet exemple le composé actif peut être remplacé par 0,0005g de l'acide p-(7-tert-butyl-6-méthoxy-2-naphtyl)-benzoïque.

| 3) Crème huile dans l'eau non-ionique | |
|---|---|
| 5-(5,6,7,8-tétrahydro-5,5,8,8-tétraméthyl-2-anthracényl)-2-furannecarboxylate de 2-hydroxyéthyle | 0,001g |
| Alcool cétylique | 3,00g |
| Alcool stéarique | 3,400g |
| Alcool cétylique oxyéthyléné (20 moles) | 0,630g |
| Alcool stéarylique oxyéthyléné (20 moles) | 1,470g |
| Monostéarate de glycérol | 2,000g |
| Huile de vaseline | 15,000g |
| Glycérine | 10,000g |
| Conservateurs | 0,050g |
| Eau distillée QSP | 100,000g |

| 4) Crème huile dans l'eau anionique | |
|---|---|
| Ethylamide de l'acide 5-(5,6,7,8-tétrahydro-5,5,8,8-tétraméthyl-2-anthracényl)-2 furannecarboxylique | 0,002g |
| Dodécyl sulfate de sodium | 0,008g |
| GLycérol | 2,000g |
| Alcool stéarylique | 20,000g |
| Triglycérides d'acide caprique/caprylique vendus par la Société DYNAMIT NOBEL sous le nom de "MIGLYOL 812" | 20,000g |
| Conservateurs | 0,050g |
| Eau déminéralisée | 100,000g |

COMPOSITIONS COSMETIQUES

| 1. Lotion anhydre | |
|---|---|
| Ester méthylique de l'acide p-[7-(1-adamantyl)-6-méthoxy-2-naphtyl]-benzoïque | 0,001g |
| Ethanol absolu | 30g |
| Polyéthylèneglycol qsp | 100g |

| 2. Gel anhydre | |
|---|---|
| Acide p-[7-(1-adamantyl)-6-méthoxy-2-naphtyl]-benzoïque | 0,001g |
| Monoéthyléther du diéthylène glycol | 35g |
| Hydroxypropylcellulose | 1g |
| Conservateurs qs Polyéthylèneglycol qsp | 100g |

| 3. Huile de bain | |
|---|---|
| Ester méthylique de l'acide p-[7-(1-adamantyl)-6-méthoxy-2-naphtyl]-benzoïque | 0,001g |
| Alcool gras éthoxylé | 10,00g |
| Octyldodécanol | 20,00g |
| Myristate d'isopropyle | 25,00g |
| Huile essentielle | 5,00g |
| Triglycérides acides $C_8$-$C_{10}$ qsp | 100g |

| 4. Stick (non soluble) | |
|---|---|
| Acide p-[7-(1-adamantyl)-6-méthoxy-2-naphtyl]-benzoïque | 0,001g |
| Beurre de cacao | 12,50g |
| Cire d'ozokérite | 18,50g |
| Paraffine dure (point de goutte: 58 ° C) | 6,25g |
| Vaseline blanche | 12,75g |
| Myristate d'isopropyle qsp | 100,00g |

| 5. Shampooing gel | |
|---|---|
| 5-(5,6,7,8-tétrahydro-5,5,8,8-tétraméthyl-2-anthracényl)-2-furanne-carboxylate de 2-hydroxyéthyle | 0,002g |
| Lauryl sulfate de sodium | 50,00g |
| Bétaïne de coco | 20,00g |
| Conservateurs qs Colorants qs Parfum qs Eau qsp | 100,00g |

| 6. Shampooing moyennement visqueux | |
|---|---|
| 5-(5,6,7,8-tétrahydro-5,5,8,8-tétraméthyl-2-anthracényl)-2-furanne-carboxylate de 2-hydroxyéthyle | 0,002g |
| Lauryl éther sulfate de sodium | 40g |
| Diéthanolamide de l'acide gras de coprah | 3,00g |
| Chlorure de sodium | 2,00g |
| Conservateurs qs Colorants qs Parfum qs Eau qsp | 100,00g |

## Revendications

**1.** Composés aromatiques polycycliques, caractérisés par le fait qu'ils répondent à la formule générale suivante :

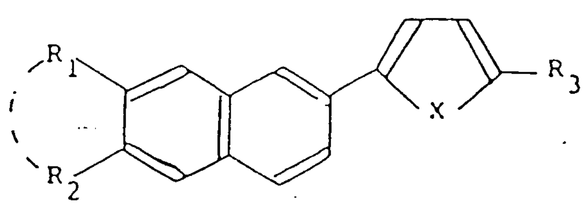

(I)

dans laquelle :

X représente -CH = CH-, 0 ou S,

$R_1$ et $R_2$, identiques ou différents, représentent un atome d'hydrogène, un radical alkyle, linéaire ou ramifié, ayant de 1 à 15 atomes de carbone, un radical alkoxy ayant de 1 à 6 atomes de carbone, un

radical 1-adamantyle, ou $R_1$ et $R_2$, pris ensemble, forment avec les atomes de carbone adjacents du noyau naphtalénique, un cycle à 5 ou 6 chaînons éventuellement substitués(s) par au moins un radical alkyle inférieur, ou interrompu par un atome d'oxygène,

$R_3$ représente le radical $-CH_2OH$, ou $-COR_4$ ou encore le radical $-CH_3$ lorsque $R_1$ et $R_2$, pris ensemble, forment un cycle à 5 ou 6 chaînons,

$R_4$ représentant

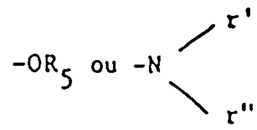

$R_5$ représentant un atome d'hydrogène, un radical alkyle ayant 1 à 20 atomes de carbone, monohydroxyalkyle, polyhydroxyalkyle, aryle ou aralkyle ou un reste d'un sucre ou encore représente le radical :

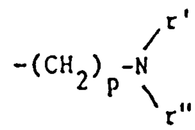

p étant 1, 2 ou 3,

r' et r'' représentant un atome d'hydrogène, un radical alkyle inférieur, un radical monohydroxyalkyle ou polyhydroxyalkyle, un radical aryle éventuellement substitue par le groupe hydroxy ou un reste d'aminoacide ou de sucre aminé ou encore pris ensemble forment un hétérocycle,

ou encore X, $R_1$ et $R_2$ représentent respectivement -CH=CH-, 1-adamantyle et -OH, et $R_3$ représente -COOH ou $-COOCH_3$

sous réserve que $R_1$ et $R_2$ ne peuvent simultanément représenter un atome d'hydrogène lorsque X représente un atome de soufre et $R_3$ le radical $-COOCH_3$,

et les sels desdits dérivés aromatiques polycycliques de formule (I).

**2.** Composés selon la revendication 1, caractérisés par le fait que le radical alkyle ayant de 1 à 20 atomes de carbone est un radical méthyle, éthyle, propyle, 2-éthyl hexyle, octyle, dodécyle, hexadécyle ou octadécyle.

**3.** Composés selon la revendication 1, caractérisés par le fait que le radical monohydroxyalkyle est un radical 2-hydroxy éthyle ou 2-hydroxypropyle.

**4.** Composés selon la revendication 1, caractérisés par le fait que le radical polyhydroxyalkyle est un radical 2,3-dihydroxy propyle, 2,3,4-trihydroxy butyle, 2,3,4,5-tétrahydroxy pentyle ou le reste du pentaérythritol.

**5.** Composés selon la revendication 1, caractérisés par le fait que le reste d'un sucre est un reste dérivant du glucose, du mannose, de l'érythrose ou du galactose.

**6.** Composés selon la revendication 1, caractérisés par le fait que le reste d'un sucre aminé est un reste dérivant de glucosamine, de galactosamine ou de mannosamine.

**7.** Composés selon la revendication 1, caractérisés par le fait que les radicaux r' et r'', pris ensemble, forment un hétérocycle pris dans le groupe constitué par un radical pipéridino, pipérazino, morpholino et pyrrolidino.

**8.** Composés selon l'une quelconque des revendications 1 et 2 caractérisés par le fait qu'ils répondent à la formule suivante :

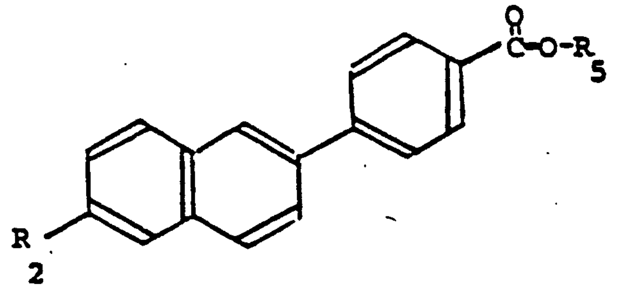

(II)

dans laquelle :

$R_5$ représente un atome d'hydrogène ou un radical alkyle,

et

$R_2$ représente un radical alkyle inférieur ramifié.

**9.** Composés selon la revendication 8 caractérisés par le fait que le radical $R_2$ est le radical tertiobutyle.

**10.** Composés selon l'une quelconque des revendications 1 et 2 caractérisés par le fait qu'ils répondent à la formule suivante :

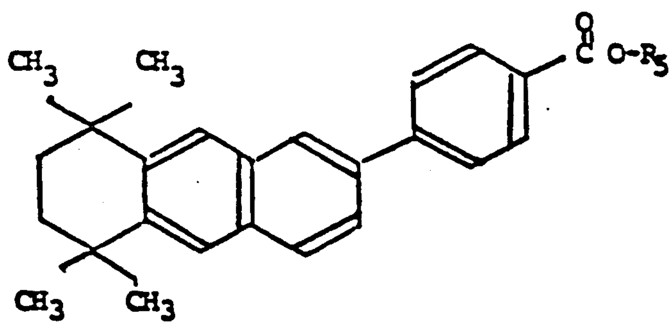

(III)

dans laquelle :

$R_5$ représente un atome d'hydrogène ou un radical alkyle

**11.** Composés selon l'une quelconque des revendications 1 et 2, caractérisés par le fait qu'ils répondent à la formule suivante:

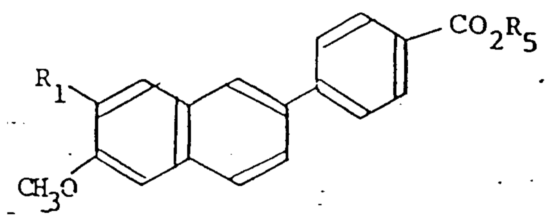

dans laquelle:

$R_1$ représente un radical tert-butyle ou 1-adamantyle,

et

$R_5$ représente un atome d'hydrogène ou un radical alkyle.

**12.** Composés selon l'une quelconque des revendications 1 et 2, caractérisés par le fait qu'ils répondent à la formule suivante:

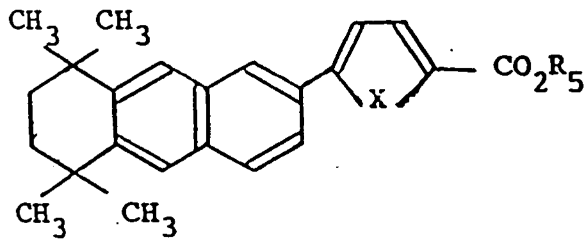

dans laquelle:
X est 0 ou S
et
$R_5$ représente un atome d'hydrogène ou un radical alkyle.

**13.** Composés selon l'une quelconque des revendications 1 à 7, caractérisés par le fait qu'ils sont pris dans le groupe constitué par :
l'acide p-(6-tert-butyl-2-naphtyl)benzoïque,
le diéthylamide de l'acide p-(6-tert-butyl-2-naphtyl)benzoïque,
l'acide p-(6-méthoxy-2-naphtyl) benzoïque et son ester méthylique,
l'acide p-[7-(1-adamantyl)-6-methoxy-2-naphtyl] benzoïque et son ester méthylique,
l'acide p-(7-tert-butyl-6-méthoxy-2-naphtyl) benzoïque et son ester méthylique, et
l'acide p-[7-(1-adamantyl)-6-hydroxy-2-naphtyl] benzoïque et son ester méthylique.

**14.** Composés selon l'une quelconque des revendications 1 à 7, caractérisés par le fait qu'ils sont pris dans le groupe constitué par :
l'acide p-(5,6,7,8-tétrahydro-5,5,8,8 tétraméthyl-2-anthracényl) benzoïque et ses esters éthylique, 2-hydroxyéthylique et 2,3-dihydroxypropylique,
le monoéthylamide de l'acide p-(5,6,7,8-tétrahydro-5,5,8,8 tétraméthyl-2-anthracényl) benzoïque,
l'alcool p-(5,6,7,8-tétrahydro-5,5,8,8-tétraméthyl-2-anthracényl) benzylique,
le 2-(p-méthylphényl)-5,6,7,8-tétrahydro-5,5,8,8-tétraméthyl anthracène, et
le p-hydroxyphénylamide de l'acide p-(5,6,7,8-tétrahydro-5,5,8,8-tétraméthyl-2-anthracényl)-benzoïque.

**15.** Composés selon l'une quelconque des revendications 1 à 7, caractérisés par le fait qu'ils sont pris dans le groupe constitué par :
l'acide 5-(5,6,7,8-tétrahydro-5,5,8,8-tétraméthyl-2-anthracényl)-2-furanne carboxylique et son ester méthylique,
l'acide 5-(5,6,7,8-tétrahydro-5,5,8,8-tétraméthyl-2-anthracényl)-2-thiophène carboxylique et son ester méthylique,
l'éthylamide de l'acide 5-(5,6,7,8-tétrahydro-5,5,8,8-tétraméthyl -2-anthracényl)-2-furanne carboxylique,
le morpholide de l'acide 5-(5,6,7,8-tétrahydro-5,5,8,8-tétraméthyl -2-anthracényl)-2-furanne carboxylique,
le 5-(5,6,7,8-tétrahydro-5,5,8,8-tétraméthyl-2-anthracényl)-2-furanne carboxylate de 2-hydroxyéthyle, et
l'acide p-[3,4(2H)-dihydro-4,4-diméthyl-7 naphto(2,3-b) pyrannyl] -benzoïque et son ester méthylique.

**16.** Procédé de préparation des composés de formule (I), caractérisé par le fait qu'il comprend les étapes consistant :
1°) à faire réagir par réaction de couplage du type Wittig ou Wittig-Horner un aldéhyde aromatique de formule :

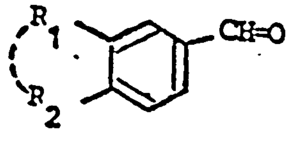

$R_1$ et $R_2$ ayant les mêmes significations que celles données à la revendication 1, avec un dérivé de phosphore pentavalent de formule :

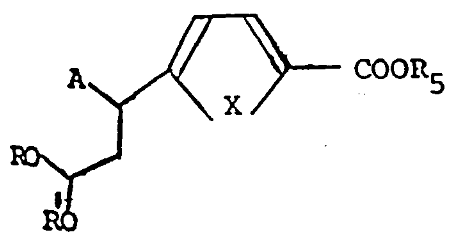

dans laquelle :

X et $R_5$ ont les mêmes significations qu'à la revendication 1,

A représente (i) soit le groupement - $P[X']_3^{\oplus}$ $Y^{\ominus}$

X' étant un aryle et Y un anion d'un acide organique ou inorganique (ii) soit le groupement -

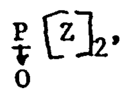

Z étant un alkoxy, et

R et R' représentent un radical alkyle inférieur ou pris ensemble forment un cycle dioxanne ou dioxolane,

et

2°) à procéder à une réaction de cyclisation-aromatisation du produit intermédiaire obtenu, dans un solvant chloré, en présence d'un catalyseur acide, ladite réaction étant éventuellement conduite sous irradiation UV.

**17.** Procédé selon la revendication 16, caractérisé par le fait que A représente le groupement

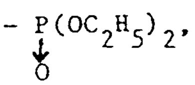

la réaction de couplage étant effectuée en présence de diisopropylamidure de lithium dans le tétrahydrofuranne à une température d'environ -80°C.

**18.** Procédé selon la revendication 16, caractérisé par le fait que la réaction de cyclisation-aromatisation est réalisée dans le dichlorométhane ou le chloroforme en présence d'acide sulfurique, d'acide paratoluène sulfonique ou de trifluorométhane sulfonate de triméthylsilyle.

**19.** Procédé de préparation des composés de formule (I), caractérisé par le fait qu'il comprend les étapes consistant:

1°) à faire réagir par réaction du type Wittig ou Wittig- Horner un aldéhyde aromatique de formule:

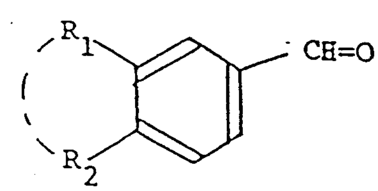

avec un dérivé de phosphore pentavalent de formule:

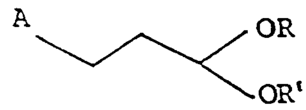

dans laquelle:

$R_1$, $R_2$, A, R et R' ont les mêmes significations qu'à la revendication 16,

2°) à faire réagir le composé intermédiaire obtenu de formule:

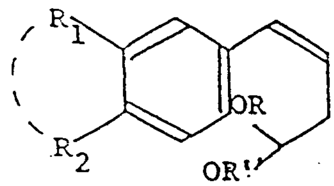

avec un halogéno-ester de formule:

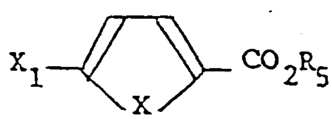

X et $R_5$ ayant les mêmes significations qu'à la revendication 1, et $X_1$ représentant Cl ou Br

3°) à procéder à une réaction de cyclisation-aromatisation du produit obtenu.

**20.** Composés intermédiaires de synthèse, caractérisés par le fait qu'ils répondent à la formule suivante:

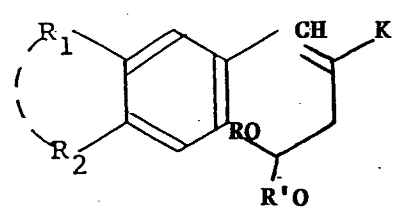

dans laquelle:

$R_1$ et $R_2$ ont les mêmes significations que celles données à la revendication 1,

K représentant un atome d'hydrogène ou le radical:

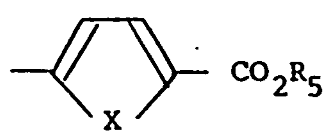

X et $R_5$ ayant les mêmes significations que celles données à la revendication 1, et R et R' représentant un radical alkyle inférieur ou pris ensemble forment a cycle dioxanne ou dioxolane.

**21.** Médicament caractérisé par le fait qu'il est un composé actif de formule (I) ou un de ses sels selon l'une quelconque des revendications 1 à 15.

**22.** Composition pharmaceutique caractérisée par le fait qu'elle contient, dans un véhicule approprié, pour une administration par voie entérale, parentérale, topique ou oculaire, au moins un composé actif de formule (I), ou a de ses sels, selon l'une quelconque des revendications 1 à 15.

**23.** Composition selon la revendication 22, caractérisée par le fait qu'elle contient de 0,00001 à environ 5%

en poids du composé actif.

**24.** Composition cosmétique pour l'hygiène corporelle et capillaire, caractérisée par le fait qu'elle contient, dans un véhicule cosmétique approprié, au moins un composé actif de formule (I) ou un de ses sels selon l'une quelconque des revendications 1 à 15.

**25.** Composition cosmétique selon la revendication 24, caractérisée par le fait qu'elle contient de 0,00001 à 2%, et de préférence entre 0,0001 et 1% en poids du composé actif.

## Claims

**1.** Polycyclic aromatic compounds, characterised in that they correspond to the following general formula:

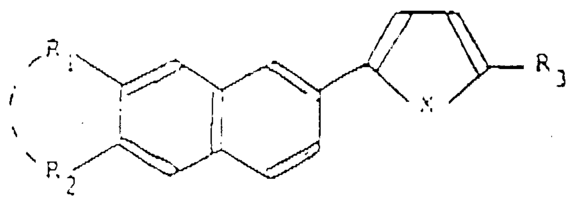

(I)

in which:

X denotes -CH=CH-, 0 or S,

$R_1$ and $R_2$, which may be identical or different, denote a hydrogen atom, a linear or branched alkyl radical having from 1 to 15 carbon atoms, an alkoxy radical having from 1 to 6 carbon atoms, or a 1-adamantyl radical, or $R_1$ and $R_2$, taken together, form, with the adjacent carbon atoms of the naphthalene ring system, a 5- or 6-membered ring optionally substituted with at least one lower alkyl radical, or interrupted by an oxygen atom, and

$R_3$ denotes a radical $-CH_2OH$ or $-COR_4$ or alternatively a $-CH_3$ radical when $R_1$ and $R_2$, taken together, form a 5- or 6-membered ring,

$R_4$ denoting $-OR_5$ or

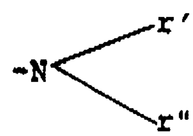

$R_5$ denoting a hydrogen atom, an alkyl radical having 1 to 20 carbon atoms, a monohydroxyalkyl, polyhydroxyalkyl, aryl or aralkyl radical, or a sugar residue, or alternatively denotes a radical:

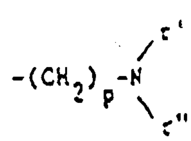

p being 1, 2 or 3,

r' and r'' denoting a hydrogen atom, a lower alkyl radical, a monohydroxyalkyl or polyhydroxyalkyl radical, an aryl radical optionally substituted with a hydroxyl group, or an amino acid residue or amino sugar residue, or alternatively, taken together, form a heterocycle, or alternatively X, $R_1$ and $R_2$ denote -CH=CH-, 1-adamantyl and -OH, respectively, and $R_3$ denotes -COOH or $-COOCH_3$ with the proviso that $R_1$ and $R_2$ cannot simultaneously denote a hydrogen atom when X denotes a sulphur atom and $R_3$ a $-COOCH_3$ radical,

and the salts of the said polycyclic aromatic derivatives of formula (I).

**2.** Compounds according to Claim 1, characterised in that the alkyl radical having from 1 to 20 carbon

atoms is a methyl, ethyl, propyl, 2-ethylhexyl, octyl, dodecyl, hexadecyl or octadecyl radical.

**3.** Compounds according to Claim 1, characterised in that the monohydroxyalkyl radical is a 2-hydroxyethyl or 2-hydroxypropyl radical.

**4.** Compounds according to Claim 1, characterised in that the polyhydroxyalkyl radical is a 2,3-dihydroxypropyl, 2,3,4-trihydroxybutyl or 2,3,4,5-tetrahydroxypentyl radical or a pentaerythritol residue.

**5.** Compounds according to Claim 1, characterised in that the sugar residue is a residue derived from glucose, from mannose, from erythrose or from galactose.

**6.** Compounds according to Claim 1, characterised in that the amino sugar residue is a residue derived from glucosamine, galactosamine or mannosamine.

**7.** Compounds according to Claim 1, characterised in that the radicale r' and r", taken together, form a heterocycle selected from the group consisting of a piperidino, piperazino, morpholino and pyrrolidino radical.

**8.** Compounds according to either of Claims 1 and 2, characterised in that they correspond to the following formula:

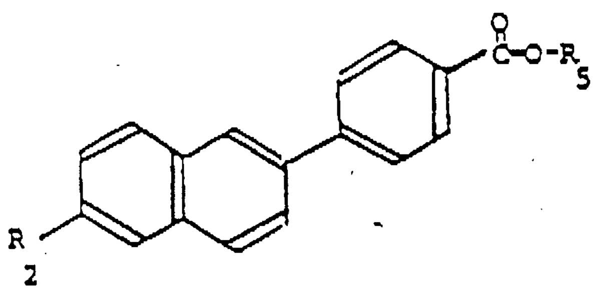

(II)

in which:
$R_5$ denotes a hydrogen atom or an alkyl radical,
and
$R_2$ denotes a branched lower alkyl radical.

**9.** Compounds according to Claim 8, characterised in that the radical $R_2$ is a tert-butyl radical.

**10.** Compounds according to either of Claims 1 and 2, characterised in that they correspond to the following formula:

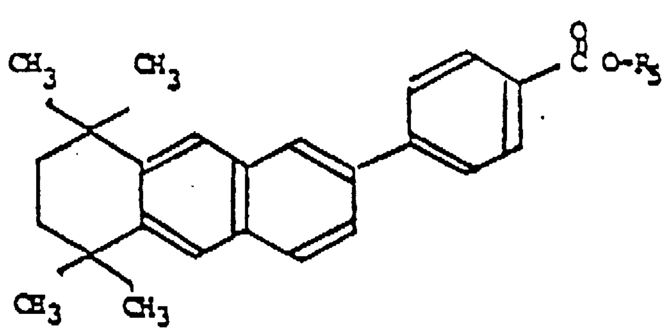

(III)

in which:
$R_5$ denotes a hydrogen atom or an alkyl radical.

**11.** Compounds according to either of Claims 1 and 2, characterised in that they correspond to the

following formula:

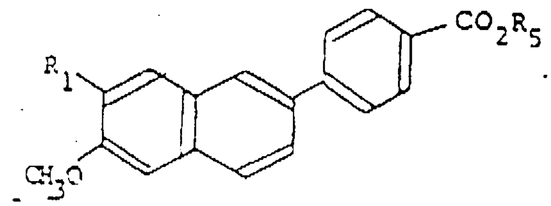

in which:

$R_1$ denotes a tert-butyl or 1-adamantyl radical,

and

$R_5$ denotes a hydrogen atom or an alkyl radical.

**12.** Compounds according to either of Claims 1 and 2, characterised in that they correspond to the following formula:

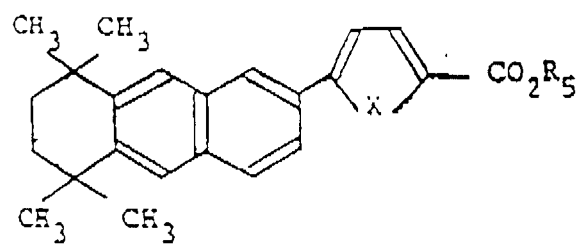

in which:

X is 0 or S

and

$R_5$ denotes a hydrogen atom or an alkyl radical.

**13.** Compounds according to any one of Claims 1 to 7, characterised in that they are selected from the group consisting of:

p-(6-tert-butyl-2-naphthyl)benzoic acid,
p-(6-tert-butyl-2-naphthyl)benzoic acid diethylamide,
p-(6-methoxy-2-naphthyl)benzoic acid and its methyl ester,
p-[7-(1-adamantyl)-6-methoxy-2-naphthyl]benzoic acid and its methyl ester,
p-(7-tert-butyl-6-methoxy-2-naphthyl)benzoic acid and its methyl ester, and
p-[7-(1-adamantyl)-6-hydroxy-2-naphthyl]benzoic acid and its methyl ester.

**14.** Compounds according to any one of Claims 1 to 7, characterised in that they are selected from the group consisting of:

p-(5,6,7,8-tetrahydro-5,5,8,8-tetramethyl-2-anthracenyl)benzoic acid and its ethyl, 2-hydroxyethyl and 2,3-dihydroxypropyl esters,
p-(5,6,7,8-tetrahydro-5,5,8,8-tetramethyl-2-anthracenyl)benzoic acid monoethylamide,
p-(5,6,7,8-tetrahydro-5,5,8,8-tetramethyl-2-anthracenyl)benzyl alcohol,
2-(p-methylphenyl)-5,6,7,8-tetrahydro-5,5,8,8-tetramethylanthracene, and
p-(5,6,7,8-tetrahydro-5,5,8,8-tetramethyl-2-anthracenyl)benzoic acid p-hydroxyphenylamide.

**15.** Compounds according to any one of Claims 1 to 7, characterised in that they are selected from the group consisting of:

5-(5,6,7,8-tetrahydro-5,5,8,8-tetramethyl-2-anthracenyl)-2-furancarboxylic acid and its methyl ester,
5-(5,6,7,8-tetrahydro-5,5,8,8-tetramethyl-2-anthracenyl)-2-thiophenecarboxylic acid and its methyl ester,
5-(5,6,7,8-tetrahydro-5,5,8,8-tetramethyl-2-anthracenyl)-2-furancarboxylic acid ethylamide,
5-(5,6,7,8-tetrahydro-5,5,8,8-tetramethyl-2-anthracenyl)-2-furancarboxylic acid morpholide,

2-hydroxyethyl 5-(5,6,7,8-tetrahydro-5,5,8,8-tetramethyl-2-anthracenyl)-2-furancarboxylate, and p-[3,4(2H)-dihydro-4,4-dimethyl-7-naphtho[2,3-b]-pyranyl]benzoic acid and its methyl ester.

**16.** Process for preparing the compounds of formula (I), characterised in that it comprises the stages consisting in:

1) reacting, by a Wittig or Wittig-Horner type coupling reaction, an aromatic aldehyde of formula:

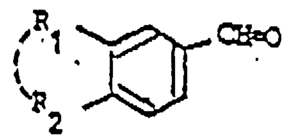

$R_1$ and $R_2$ having the same meanings as those given in Claim 1, with a pentavalent phosphorus derivative of formula:

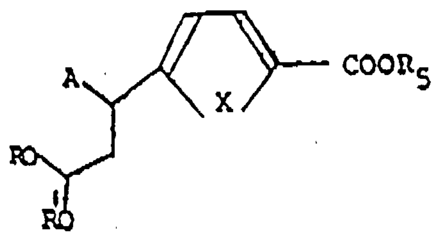

in which:

X and $R_5$ have the same meanings as in Claim 1,

A denotes (i) either a group $-P(X']_3^{\oplus}Y^{\ominus}$, X' being an aryl and Y an anion of an organic or inorganic acid, (ii) or a group

$$-\underset{\underset{O}{\downarrow}}{P}[Z]_2,$$

Z being an alkoxy, and

R and R' denote a lower alkyl radical or, taken together, form a dioxane or dioxolane ring, and 2) performing a cyclisation/aromatisation reaction of the intermediate obtained, in a chlorinated solvent, in the presence of an acid catalyst, said reaction optionally being performed under UV irradiation.

**17.** Process according to Claim 16, characterised in that A denotes a

$$-\underset{\underset{O}{\downarrow}}{P}(OC_2H_5)_2$$

group, the coupling reaction being carried out in the presence of lithium diisopropylamide in tetrahydrofuran at a temperature of approximately -80 ° C.

**18.** Process according to Claim 16, characterised in that the cyclisation/aromatisation reaction is carried out in dichloromethane or chloroform in the presence of sulphuric acid, para-toluenesulphonic acid or trimethylsilyl trifluoromethanesulphonate.

**19.** Process for preparing the compounds of formula (I), characterised in that it comprises the stages consisting in:

1) reacting, by a Wittig or Wittig-Horner type reaction, an aromatic aldehyde of formula:

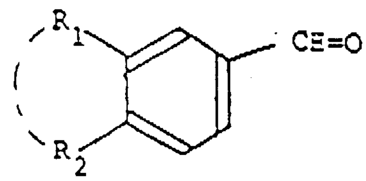

with a pentavalent phosphorus derivative of formula:

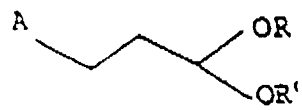

in which:

$R_1$, $R_2$, A, R and R' have the same meanings as in Claim 16,

2) reacting the intermediate compound obtained, of formula

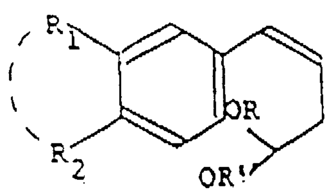

with a halo ester of formula:

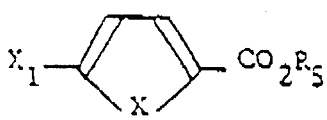

X and $R_5$ having the same meanings as in Claim 1, and

$X_1$ denoting Cl or Br,

and

3) performing a cyclisation/aromatisation reaction of the product obtained.

**20.** Synthesis intermediate compounds, characterised in that they correspond to the following formula

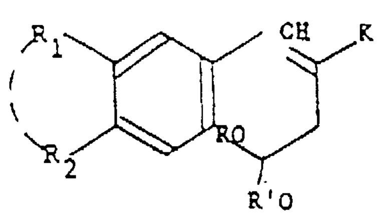

in which:

$R_1$ and $R_2$ have the same meanings as those given in Claim 1,

K denoting a hydrogen atom or a radical:

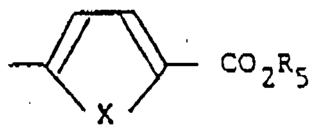

X and $R_5$ having the same meanings as those given in Claim 1, and R and R' denoting a lower alkyl radical or, taken together, form a dioxane or dioxolane ring.

**21.** Medicinal product, characterised in that it is an active compound of formula (I) or one of its salts according to any one of Claims 1 to 15.

**22.** Pharmaceutical composition, characterised in that it contains, in a vehicle suitable for enteral, parenteral, topical or ocular administration, at least one active compound of formula (I), or one of its salts, according to any one of Claims 1 to 15.

**23.** Composition according to Claim 22, characterised in that it contains from 0.00001 to approximately 5% by weight of the active compound.

**24.** Cosmetic composition for body and hair hygiene, characterised in that it contains, in a suitable cosmetic vehicle, at least one active compound of formula (I) or one of its salts according to any one of Claims 1 to 15.

**25.** Cosmetic composition according to Claim 24, characterised in that it contains from 0.00001 to 2%, and preferably between 0.0001 and 1%, by weight of the active compound.

**Patentansprüche**

**1.** Aromatische polycyclische Verbindungen, gekennzeichnet durch die folgende allgemeine Formel:

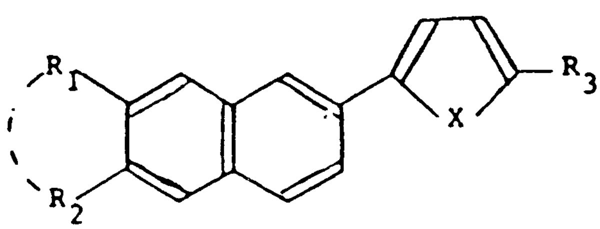

(I)

worin

X für -CH=CH-, 0 oder S steht,

$R_1$ und $R_2$ gleich oder verschieden sind und für ein Wasserstoffatom, einen geradkettigen oder verzweigten Alkylrest mit 1 bis 15 Kohlenstoffatomen, einen Alkoxyrest mit 1 bis 6 Kohlenstoffatomen oder einen 1-Adamantylrest stehen, oder $R_1$ und $R_2$ zusammen mit den angrenzenden Kohlenstoffatomen am Naphthalinkern einen 5- oder 6-gliedrigen Ring bilden, der gegebenenfalls durch mindestens einen Niedrigalkylrest substituiert oder durch ein Sauerstoffatom unterbrochen ist,

$R_3$ für den $-CH_2OH$- oder $-COR_4$-
Rest oder den $-CH_3$- Rest steht, wenn $R_1$ und $R_2$ zusammen einen 5- oder 6-gliedrigen Ring bilden,
wobei

$R_4$ für $-OR_5$ oder

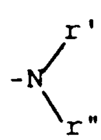

steht, und

$R_5$ für ein Wasserstoffatom, einen Alkylrest mit 1 bis 20 Kohlenstoffatomen, einen Monohydroxyalkylrest, Polyhydroxyalkylrest, einen Arylrest oder Aralkylrest oder für einen Rest eines Zuckers steht, oder einen

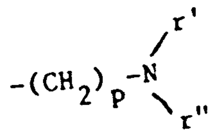

-Rest bedeutet,
wobei

p 1, 2 oder 3 bedeutet und

r' und r" für ein Wasserstoffatom, einen Niedrigalkylrest, einen Mono- oder Polyhydroxyalkylrest, einen gegebenenfalls durch eine Hydroxylgruppe substituierten Arylrest oder einen Rest einer Aminosäure oder eines aminierten Zuckers stehen oder zusammen einen Heterozyklus bilden,

oder

X, $R_1$ und $R_2$ für -C = C-,
1-Adamantyl bzw. -OH stehen und $R_3$ für -COOH oder $-COOCH_3$ steht,
mit der Maßgabe, daß $R_1$ und $R_2$ nicht gleichzeitig für ein Wasserstoffatom stehen, wenn X für ein Schwefelatom steht und $R_3$ für den Rest $-COOCH_3$ steht,

sowie Salze der aromatischen polycyclischen Derivate der Formel (I).

**2.** Verbindungen nach Anspruch 1, **dadurch gekennzeichnet**, **daß** der Alkylrest mit 1 bis 20 Kohlenstoffatomen ein Methyl-, Ethyl-, Propyl-, 2-Ethylhexyl-, Octyl-, Dodecyl-, Hexadecyl- oder Octadecylrest ist.

**3.** Verbindungen nach Anspruch 1, **dadurch gekennzeichnet, daß** der Monohydroxyalkylrest ein 2-Hydroxyethyl- oder ein 2-Hydroxypropylrest ist.

**4.** Verbindungen nach Anspruch 1, **dadurch gekennzeichnet, daß** der Polyhydroxyalkylrest ein 2,3-Dihydroxypropyl-, 2,3,4-Trihydroxybutyl-, 2,3,4,5-Tetrahydroxypentylrest oder Pentaerythritolrest ist.

**5.** Verbindungen nach Anspruch 1, **dadurch gekennzeichnet, daß** der Rest eines Zuckers ein von Glucose, Mannose, Erythrose oder Galactose abgeleiteter Rest ist.

**6.** Verbindungen nach Anspruch 1, **dadurch gekennzeichnet, daß** der Rest eines aminierten Zuckers ein von Glucosamin, Galactosamin oder Mannosamin abgeleiteter Rest ist.

**7.** Verbindugnen nach Anspruch 1, **dadurch gekennzeichnet**, **daß** die Reste r' und r" zusammen einen Heterozyklus bilden, welcher ausgewählt ist unter einem Piperidin-, Piperazin-, Morpholin- und Pyrrolidinrest.

**8.** Verbindungen nach einem der Ansprüche 1 und 2, **gekennzeichnet durch** die folgende Formel:

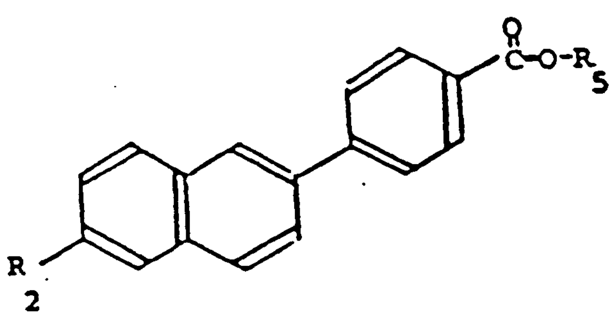

**(II)**

worin
$R_5$ für ein Wasserstoffatom oder einen Alkylrest,
und
$R_2$ für einen verzweigten Niedrigalkylrest stehen.

**9.** Verbindungen nach Anspruch 8, **dadurch gekennzeichnet, daß** der $R_2$-Rest ein t-Butylrest ist.

**10.** Verbindungen nach einem der Ansprüche 1 und 2, **gekennzeichnet durch** die folgende Formel:

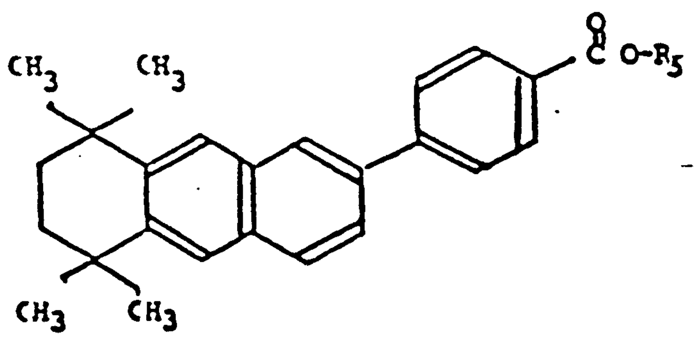

(III)

worin $R_5$ für ein Wasserstoffatom oder einen Alkylrest steht.

**11.** Verbindungen nach einem der Ansprüche 1 und 2, **gekennzeichnet durch** die folgende Formel:

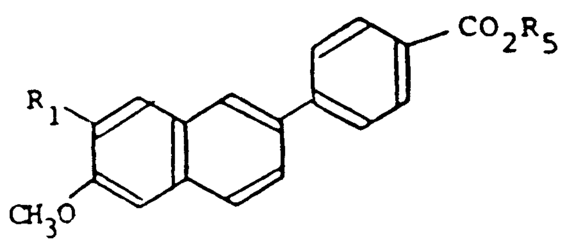

worin
$R_1$ für einen t-Butyl- oder 1-Adamantylrest und
$R_5$ für ein Wasserstoffatom oder einen Alkylrest stehen.

**12.** Verbindungen nach einem der Ansprüche 1 und 2, **gekennzeichnet durch** die folgende Formel:

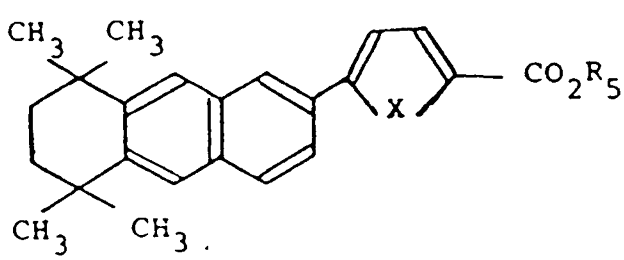

worin
X für 0 oder S und
$R_5$ für ein Wasserstoffatom oder einen Alkylrest stehen.

**13.** Verbindungen nach einem der Ansprüche 1 bis 7, **dadurch gekennzeichnet,** daß sie ausgewählt sind unter

- p-(6-t-Butyl-2-naphthyl)-benzoesäure,

- p-(6-t-Butyl-2-naphthyl)-benzoesäurediethylamid,
- p-(6-Methoxy-2-naphthyl)-benzoesäure und deren Methylester,
- p-[7-(1-Adamantyl)-6-methoxy-2-naphthyl]-benzoesäure und deren Methylester,
- p-(7-t-Butyl-6-methoxy-2-naphthyl)-benzoesäure und deren Methylester und
- p-[7-(1-Adamantyl)-6-hydroxy-2-naphthyl]-benzoesäure und deren Methylester.

**14.** Verbindungen nach einem der Ansprüche 1 bis 7, **dadurch gekennzeichnet**, **daß** sie ausgewählt sind unter
- p-(5,6,7,8-Tetrahydro-5,5,8,8-tetramethyl-2-anthracenyl)-benzoesäure und deren Ethyl-, 2-Hydroxyethyl- und 2,3-Dihydroxypropylester,
- p-(5,6,7,8-Tetrahydro-5,5,8,8-tetramethyl-2-anthracenyl)-benzoesäuremonoethylamid,
- p-(5,6,7,8-Tetrahydro-5,5,8,8-tetramethyl-2-anthracenyl)-benzylalkohol,
- 2-(p-Methylphenyl)-5,6,7,8-tetrahydro-5,5,8,8-tetramethylanthracen und
- p-(5,6,7,8-Tetrahydro-5,5,8,8-tetramethyl-2-anthracenyl)-benzoesäure-p-hydroxyphenylamid,

**15.** Verbindungen nach einem der Ansprüche 1 bis 7, **dadurch gekennzeichnet,** daß sie ausgewählt sind unter
- 5-(5,6,7,8-Tetrahydro-5,5,8,8-Tetramethyl-2-anthracenyl)-2-furancarbonsäure und deren Methylester,
- 5-(5,6,7,8-Tetrahydro-5,5,8,8-Tetramethyl-2-anthracenyl)-2-thiophencarbonsäure und deren Methylester,
- 5-(5,6,7,8-Tetrahydro-5,5,8,8-tetramethyl-2-anthracenyl)-2-furancarbonsäureethylamid,
- 5-(5,6,7,8-Tetrahydro-5,5,8,8-tetramethyl-2-anthracenyl)-2-furancarbonsäuremorpholid,
- 2-Hydroxyethyl-5-(5,6,7,8-tetrahydro-5,5,8,8-tetramethyl-2-anthracenyl)-2-furancarboxylat und
- p-[3,4(2H)-Dihydro-4,4-dimethyl-7-naphtho-(2,3-b)-pyranyl]-benzoesäure und deren Methylester.

**16.** Verfahren zur Herstellung der Verbindungen der Formel (I), **dadurch gekennzeichnet,** daß es folgende Stufen umfaßt:

1.) Umsetzung eines aromatischen Aldehyds der Formel

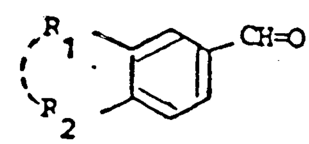

worin

$R_1$ und $R_2$ die gleichen Bedeutungen wie in Anspruch 1 besitzen, in einer Wittig- oder Wittig-Horner-Kopplungsreaktion

mit einem fünfwertigen Phosphorderivat der Formel

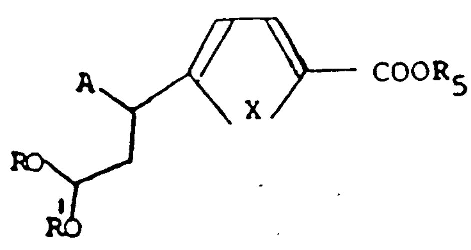

worin

| | |
|---|---|
| X und $R_5$ | die gleichen Bedeutungen wie in Anspruch 1 besitzen, |
| A | (i) entweder für die - $P[X']_3^{\oplus}$ $Y^{\ominus}$ -Gruppe steht, worin X' ein Aryl und Y ein Anion einer organischen oder anorganischen Säure bedeuten, oder (ii) für die |

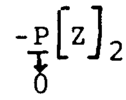

-Gruppe steht,
worin Z ein Alkoxy bedeutet,
und

R und R' für einen Niedrigalkylrest stehen oder zusammen einen Dioxan- oder Dioxolanzyklus bilden,

und

2.) Zyklisierung des erhaltenen Zwischenprodukts in einem chlorierten Lösungsmittel in Anwesenheit eines sauren Katalysators unter Bildung eines Aromaten, wobei die Reaktion gegebenenfalls unter UV-Bestrahlung durchgeführt wird.

**17.** Verfahren nach Anspruch 16, **dadurch gekennzeichnet,** daß A für die Gruppe

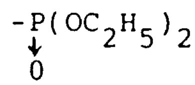

steht und die Reaktion bei einer Temperatur von ca. - 80 ° C in Anwesenheit von Lithiumdiisopropylamid in Tetrahydrofuran durchgeführt wird.

**18.** Verfahren nach Anspruch 16, **dadurch gekennzeichnet,** daß die Zyklisierung unter Bildung eines Aromaten in Dichlormethan oder Chloroform in Anwesenheit von Schwefelsäure, p-Toluolsulfonsäure oder Trimethylsilyltrifluormethansulfonat durchgeführt wird.

**19.** Verfahren zur Herstellung der Verbindungen der Formel (I), **dadurch gekennzeichnet**, daß es folgende Stufen umfaßt:

1.) Umsetzen eines aromatischen Aldehyds der Formel:

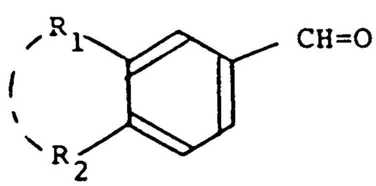

in einer Wittig- oder Wittig-Horner-Reaktion mit einem fünfwertigen Phosphorderivat der Formel:

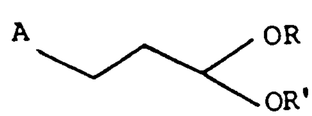

worin $R_1$, $R_2$, A, R und R' die gleichen Bedeutungen wie in Anspruch 16 besitzen,

2.) Umsetzen des erhaltenen Zwischenprodukts der Formel:

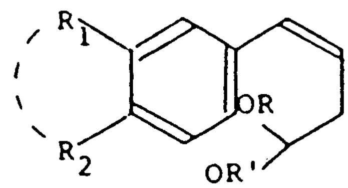

mit einem Halogenester der Formel:

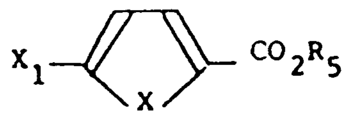

worin X und $R_5$ die gleichen Bedeutungen wie in Anspruch 1 besitzen und $X_1$ für Cl oder Br steht, und

3.) Durchführung einer Zyklisierung des erhaltenen Produktes unter Bildung eines Aromaten.

**20.** Zwischenverbindungen, **gekennzeichnet durch** folgende Formel:

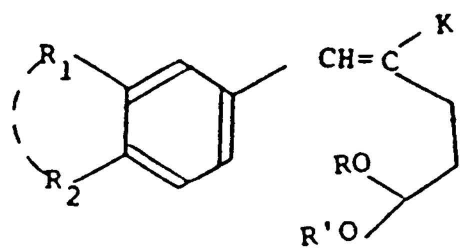

worin

| | |
|---|---|
| $R_1$ und $R_2$ | die gleichen Bedeutungen wie in Anspruch 1 besitzen, |
| K | für ein Wasserstoffatom oder den Rest 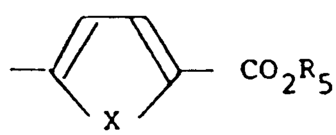 steht, worin X und $R_5$ die gleichen Bedeutungen wie in Anspruch 1 besitzen, und |
| R und R' | für einen Niedrigalkylrest stehen oder zusammen einen Dioxan- oder Dioxalanzyklus bilden. |

**21.** Medikament, **dadurch gekennzeichnet**, daß es sich dabei um eine aktive Verbindung der Formel (I) oder eines der Salze davon nach einem der Ansprüche 1 bis 15 handelt.

**22.** Pharmazeutisches Mittel, **dadurch gekennzeichnet,** daß es in einem zur enteralen, parenteralen, topischen oder okularen Verabreichung geeigneten Träger mindestens eine aktive Verbindung der Formel (I) oder eines der Salze davon nach einem der Ansprüche 1 bis 15 enthält.

**23.** Mittel nach Anspruch 22, **dadurch gekennzeichnet,** daß es 0,00001 bis ca. 5 Gew.-% der aktiven Verbindung enthält.

24. Kosmetisches Mittel zur Körper- und Haarhygiene, **dadurch gekennzeichnet,** daß es in einem geeigneten kosmetischen Träger mindestens eine aktive Verbindung der Formel (I) oder eines der Salze davon nach einem der Ansprüche 1 bis 15 enthält.

25. Kosmetisches Mittel noch Anspruch 24, **dadurch gekennzeichnet,** daß es 0,00001 bis 2 Gew.-%, vorzugsweise 0,0001 bis 1 Gew.-%, der aktiven Verbindung enthält.